# EUROPEAN PATENT APPLICATION

(11) **EP 4 257 052 A1**
(43) Date of publication of application: **11.10.2023**
(21) Application number: 21903294.3
(22) Date of filing: 02.12.2021
(51) Int. Cl.: A61B 7/04, G16H 50/30

(54) **INFORMATION PROCESSING DEVICE, INFORMATION PROCESSING METHOD, AND RECORDING MEDIUM**

(30) Priority: 07.12.2020 JP 2020202488
(71) Applicant: Suntory Holdings Limited, Osaka 530-8203 (JP)
(72) Inventor: KANEGAWA, Norimasa, Kyoto 6190284 (JP); KUTSUMI, Yuka, Kyoto 6190284 (JP); ZEIDA, Mitsuhiro, Kyoto 6190284 (JP)
(74) Representative: Vossius & Partner Patentanwälte Rechtsanwälte mbB
(86) International application number: PCT/JP2021/044234
(87) International publication number: WO 2022/124190

(57) **Abstract**

The present invention solves a problem that it is difficult for conventional information processing apparatuses to obtain objective information on the gut condition. An information processing apparatus 100 includes: a sound information acquiring unit 141 that acquires sound information regarding abdominal sounds, which are sounds emanating from the abdomen, of a user; a gut score acquiring unit 149 that acquires a gut score related to a gut condition of the user, using input information containing the sound information acquired by the sound information acquiring unit 141 and learning information prepared in advance; and a gut score output unit 161 that outputs the gut score acquired by the gut score acquiring unit 149. Accordingly, it is possible to obtain objective information on the gut condition.

## Description

### Technical Field

The present invention relates to an information processing apparatus, an information processing method, and a recording medium for making it possible to acquire information regarding the gut condition of a user.

### Background Art

In recent years, users have become increasingly aware of their own health. Under these circumstances, various devices and services have been provided to support information on health and lifestyle habits. For example, Patent Document 1 below describes the configuration of a health management support system configured to acquire and record health management information on a user and output messages pertaining to health management to the user based on the content of the information.

Furthermore, the intestines (small and large intestines) are known as one of the most important organs for digestion and absorption of nutrients and for symbiosis with gut bacteria. In recent years, the need for gut health has been increasing along with the term "gut activity" and the like.

Patent Document 2 below describes an analysis device that evaluates gastrointestinal motility for the purpose of diagnosing intestinal diseases, using acoustic feature values extracted using a machine learning method from recorded data acquired with a non-contact microphone.

### Citation List

### Patent Documents

Patent Document 1: JP 2017-041035A
Patent Document 2: WO 2019/216320

### Summary of Invention

### Technical Problem

With the growing awareness of health, matters focusing on specific organs and parts of the body may be of interest to users. For example, the condition of the guts tends to change depending on various factors such as physical condition and the contents of food and drink. Therefore, it is useful for many users as well, who are non-specialists and do not have specialized knowledge of medicine and the like, to be able to obtain objective and easily understandable information on their own gut condition.

Conventionally, in clinical practice, for example, physicians have diagnosed gut function by listening to the sound of peristalsis in the abdomen using a stethoscope and evaluating whether or not peristalsis movement is active based on this sound. With regard to the diagnosis of intestinal diseases, a device such as that described in Patent Document 2 has also been proposed. However, it was not possible for a user to obtain objective and easily understandable information on his or her own gut condition.

### Solution to Problem

A first aspect of the present invention is directed to an information processing apparatus including: a sound information acquiring unit that acquires sound information regarding abdominal sounds, which are sounds emanating from the abdomen, of a user; a gut score acquiring unit that acquires a gut score related to a gut condition of the user, using input information containing the sound information acquired by the sound information acquiring unit and learning information prepared in advance; and a gut score output unit that outputs the gut score acquired by the gut score acquiring unit.

By utilizing this configuration, it is possible for the user to obtain objective information on the gut condition.

Furthermore, a second aspect of the present invention is directed to the information processing apparatus according to the first aspect, wherein the input information further contains life information regarding a life state of the user.

By utilizing this configuration, it is possible to output more reliable information on the gut condition based on a life state of the user as well.

Furthermore, a third aspect of the present invention is directed to the information processing apparatus according to the second aspect, wherein the life information contains excretion-related information regarding an excretion status of the user.

By utilizing this configuration, it is possible to output more reliable information on the gut condition based on an excretion status of the user as well.

Furthermore, a fourth aspect of the present invention is directed to the information processing apparatus according to the third aspect, wherein the excretion-related information contains information indicated by Bristol Stool Form Scale input by the user.

By utilizing this configuration, it is easy to prepare information for obtaining more reliable information on the gut condition.

Furthermore, a fifth aspect of the present invention is directed to the information processing apparatus according to the third aspect, wherein the gut score acquiring unit further includes an excretion score acquiring unit that acquires an excretion score based on the excretion-related information, and acquires the gut score using the excretion score acquired by the excretion score acquiring unit.

By utilizing this configuration, it is possible to output a gut score reflecting an excretion status of the user.

Furthermore, a sixth aspect of the present invention is directed to the information processing apparatus according to the second aspect, wherein the life information contains eating-and-drinking information regarding an eating-and-drinking status of the user.

By utilizing this configuration, it is possible to output more reliable information on the gut condition based on an eating-and-drinking status of the user as well.

Furthermore, a seventh aspect of the present invention is directed to the information processing apparatus according to the sixth aspect, wherein the eating-and-drinking information contains at least one of information regarding the amount of water consumed, information regarding whether or not alcohol was consumed or the amount of alcohol consumed, information regarding whether or not a meal was taken or the content thereof, and information regarding whether or not a particular group of food was consumed or the amount thereof consumed.

By utilizing this configuration, it is possible to output a gut score reflecting a specific eating-and-drinking status.

Furthermore, an eighth aspect of the present invention is directed to the information processing apparatus according to the sixth aspect, wherein the gut score acquiring unit further includes an eating-and-drinking score acquiring unit that acquires an eating-and-drinking score based on the eating-and-drinking information, and acquires the gut score using the eating-and-drinking score acquired by the eating-and-drinking score acquiring unit.

By utilizing this configuration, it is possible to output a gut score reflecting an eating-and-drinking status of the user.

Furthermore, a ninth aspect of the present invention is directed to the information processing apparatus according to the second aspect, wherein the life information contains activity status information regarding an activity status of the user.

By utilizing this configuration, it is possible to output more reliable information on the gut condition based on an activity status of the user as well.

Furthermore, a tenth aspect of the present invention is directed to the information processing apparatus according to the ninth aspect, wherein the activity status information contains at least one of sleep information regarding sleep and exercise information regarding exercise.

By utilizing this configuration, it is possible to output a gut score reflecting a sleep status or an exercise status.

Furthermore, an eleventh aspect of the present invention is directed to the information processing apparatus according to the ninth aspect, wherein the activity status information is information acquired by an activity tracker that acquires the level of activity of the user.

By utilizing this configuration, it is possible to output more reliable information on the gut condition based on quantitatively obtained activity information.

Furthermore, a twelfth aspect of the present invention is directed to the information processing apparatus according to the ninth aspect, wherein the gut score acquiring unit further includes an activity status score acquiring unit that acquires an activity status score based on the activity status information, and acquires the gut score using the activity status score acquired by the activity status score acquiring unit.

By utilizing this configuration, it is possible to output a gut score reflecting an activity status of the user.

Furthermore, a thirteenth aspect of the present invention is directed to the information processing apparatus according to the first aspect, wherein the learning information is generated such that learning input information containing sound information is taken as information that is to be input and a value of a predetermined output indicator regarding an activity state of the guts is taken as information that is to be output, and the gut score acquiring unit acquires the gut score using the value of the output indicator acquired using the learning information.

By utilizing this configuration, it is easy to output objective information on the gut condition.

Furthermore, a fourteenth aspect of the present invention is directed to the information processing apparatus according to the thirteenth aspect, wherein the output indicator is at least one of a bowel movement state and the number of peristalsis movements of the guts per unit time.

By utilizing this configuration, it is possible to accurately acquire a gut score based on a value of at least one of a bowel movement state and the number of peristalsis movements of the guts per unit time.

Furthermore, a fifteenth aspect of the present invention is directed to the information processing apparatus according to the first aspect, wherein the gut score acquiring unit includes an element score acquiring unit that acquires element scores respectively for two or more evaluation elements based on the input information, and acquires the gut score using the element scores acquired by the element score acquiring unit, and the output unit further outputs a radar chart using the element scores acquired by the element score acquiring unit.

By utilizing this configuration, it is possible to output easily understandable information on each evaluation element on the gut condition.

Furthermore, a sixteenth aspect of the present invention is directed to the information processing apparatus according to the first aspect, further including: a device identifying information acquiring unit that acquires device identifying information for identifying the type of device used to acquire abdominal sounds corresponding to the sound information, wherein multiple pieces of learning information are each prepared in association with device identifying information, and the gut score acquiring unit acquires the gut score using the learning information corresponding to the device identifying information acquired by the device identifying information acquiring unit.

By utilizing this configuration, it is possible to properly acquire a gut score according to the properties and the like of a device used to acquire abdominal sounds.

Furthermore, a seventeenth aspect of the present invention is directed to the information processing apparatus according to the first aspect, further including: a microphone for recording the abdominal sounds; and a display unit that displays the gut score output by the output unit.

By utilizing this configuration, it is easy for a user to measure abdominal sounds and obtain objective information on the gut condition.

### Advantageous Effects of Invention

According to the information processing apparatus and the like of the present invention, it is possible to output objective and easily understandable information on the user's own gut condition.

### Brief Description of Drawings

FIG. 1 is a diagram showing the outline of an information processing system according to an embodiment of the present invention.
FIG. 2 is a block diagram of an information processing apparatus in the embodiment.
FIG. 3 is a block diagram of a terminal apparatus in the embodiment.
FIG. 4 is a flowchart showing an example of an operation of the information processing apparatus in the embodiment.
FIG. 5 is a flowchart showing an example of score acquiring processing of the information processing apparatus in the embodiment.
FIG. 6 is a flowchart showing an example of abdominal sound usage processing of the information processing apparatus in the embodiment.
FIG. 7 is a flowchart showing an example of gut-related score acquiring processing of the information processing apparatus in the embodiment.
FIG. 8 is a flowchart showing an example of excretion score acquiring processing of the information processing apparatus in the embodiment.
FIG. 9 is a flowchart showing an example of food score acquiring processing of the information processing apparatus in the embodiment.
FIG. 10 is a flowchart showing an example of drink score acquiring processing of the information processing apparatus in the embodiment.
FIG. 11 is a flowchart showing an example of activity status score acquiring processing of the information processing apparatus in the embodiment.
FIG. 12 is a first diagram showing a specific example of screen transition of the terminal apparatus in the embodiment.
FIG. 13 is a second diagram showing a specific example of screen transition of the terminal apparatus in the embodiment.
FIG. 14 is a third diagram showing a specific example of screen transition of the terminal apparatus in the embodiment.
FIG. 15 is a fourth diagram showing a specific example of screen transition of the terminal apparatus in the embodiment.
FIG. 16 is a flowchart showing an example of abdominal sound usage processing of the information processing apparatus according to a first modified example of the embodiment of the present invention.
FIG. 17 is a flowchart showing an example of gut-related score acquiring processing of the information processing apparatus in the embodiment.
FIG. 18 is a flowchart showing an example of abdominal sound usage processing of the information processing apparatus according to a second modified example of the embodiment of the present invention.
FIG. 19 is a flowchart showing an example of score acquiring processing of the information processing apparatus according to a third modified example of the embodiment of the present invention.
FIG. 20 is a flowchart showing an example of gut-related score acquiring processing of the information processing apparatus in the embodiment.
FIG. 21 is a block diagram showing the configuration of the terminal apparatus according to another modified example of the embodiment.
FIG. 22 is a schematic view of a computer system in the embodiment.
FIG. 23 is a block diagram of the computer system in the embodiment.

### Description of Embodiment

Below, an embodiment of the information processing apparatus and the like will be described with reference to the drawings. The constituent elements denoted by the same reference numerals in the embodiments perform similar operations, and thus a description thereof may not be repeated.

The terms used below are generally defined as follows. The meanings of these terms do not always have to be interpreted as indicated herein, and have to be interpreted in light of individual explanations below, if any, for example.

The abdominal sounds refer to sounds emanating from the abdomen of a user. The abdominal sounds may include gut sounds emanating from the intestines, for example. The abdominal sounds may include sounds emanating due to blood flow in the abdomen (e.g., abdominal aortic sounds) and sounds emanating from organs such as the stomach.

The sound information refers to information obtained based on abdominal sounds. The sound information may be the recorded abdominal sound data itself, or data obtained by processing or editing the data.

The life information is information regarding the user's life state. The life state includes various elements such as those regarding the user's behavior, state, and the like. In the following embodiment, the life information includes excretion-related information, eating-and-drinking information, and activity status information, for example. That is to say, the life state includes the user's excretion status, the user's eating-and-drinking status, and the user's activity status, for example. Note that the life information is not limited to this, and may include information regarding other elements or information regarding only some of these elements. The information on each element of the life information (excretion-related information, eating-and-drinking information, activity status information, etc.) may include information on detailed elements.

The excretion-related information is information regarding the user's excretion status. The excretion-related information may include information from various viewpoints such as the time of excretion, amount, odor, and form (shape, color, etc.) of excrement. In this embodiment, the excretion-related information includes a value indicated by known Bristol Stool Form Scale (alternatively referred to as the "Bristol scale" hereinafter). The Bristol scale indicates the physiological transit time through the gastrointestinal tract and can be said to indirectly express the state of the guts. Labels corresponding to the values expressed by the Bristol scale (e.g., "banana", "sausage", "mushy", etc.) may be used as the excretion-related information. The excretion-related information may be information resulting from the evaluation of excretion by an evaluator such as a user (information involving the subjectivity of the evaluator) or information resulting from measurement or evaluation by an evaluation device or the like.

The eating-and-drinking information is information regarding the user's eating-and-drinking status. The eating-and-drinking information may include information from various viewpoints such as the amount of water consumed, the amount of alcohol consumed, and the content of a meal. The eating-and-drinking information may be conceptually divided into food information regarding meals and eating habits and drink information regarding drink consumption. In this embodiment, the eating-and-drinking information includes, but is not limited to, at least one of information regarding the amount of water consumed, information regarding whether or not alcohol was consumed or the amount of alcohol consumed, information regarding whether or not a meal was taken or the content of a meal, and information regarding whether or not a particular group of food was consumed or the amount thereof consumed. The particular group of food refers to food that belongs to a particular group among the groups classified as meat, dairy products, vegetables, and the like, for example, but the viewpoint and granularity of such "group" classification are not limited to this. The eating-and-drinking information is, but is not limited to, information that is input by an evaluator such as a user who performed evaluation of the eating-and-drinking status. For example, the eating-and-drinking information may be information resulting from measurement or evaluation by an evaluation device or the like. For example, the types and amounts of food or drink consumed may have different effects on digestive motility.

The activity status information is information regarding the user's activity status. The activity status herein refers to a status related to lifestyle habits (i.e., actions and behaviors that the user repeatedly performs in daily life) excluding the eating-and-drinking status. That is to say, the activity status information may include information regarding lifestyle habits excluding the eating-and-drinking information. In this embodiment, the activity status information includes, but is not limited to, at least one of sleep information regarding sleep (sleep duration, sleep quality, etc.) and exercise information regarding exercise (the number of steps, the amount of calories burned, whether or not a user engages in habitual exercise, intensity of exercise, frequency of exercise, etc.). For example, the activity status information may include information regarding whether or not a user smokes, smoking history, and the like. The activity status information may be information acquired by an activity tracker installed in a device (e.g., a wearable terminal or other portable terminal, etc.) that the user carries around or wears, or information input by an evaluator such as a user who performed evaluation, for example.

The basic information is information regarding the user's type and characteristics. For example, the basic information may include various types of information such as the user's height, weight, age, sex, body fat percentage, muscle mass, pulse rate, respiratory rate, blood pressure, and the like. The basic information may further include user's responses to questionnaires, information generated based on those responses, and information regarding the user's medical history. For example, responses to questionnaire items such as those used to diagnose people with intestinal disorders can objectively represent the user's physical constitution. The basic information is input by a user or the like in advance, for example, but there is no limitation to this. For example, the basic information may be information acquired by a wearable terminal or the like that the user wears, or information acquired from an external database or the like in which user information is recorded.

An identifier for a matter is a letter, a symbol, or the like for uniquely identifying the matter. The identifier is an ID, for example, but may be any type of information with which the corresponding matter can be identified. That is to say, the identifier may be the name of the matter itself that it indicates, or symbols that are combined so as to uniquely correspond to the matter.

The acquiring may include acquiring a matter that is input by a user or the like, or acquiring information stored in the apparatus or another apparatus (the information may be information stored in advance or information generated by information processing in the apparatus). The acquiring information stored in another apparatus may include acquiring information stored in the other apparatus via an API or the like, or acquiring the content of a document file provided by the other apparatus (the content includes the webpage content, etc.) through scraping or the like. The acquiring may include acquiring information in a different format based on the original information, such as acquiring information through optical character reading of an image file.

Furthermore, a so-called machine learning method may be used to acquire information. A machine learning method may be used as follows, for example. That is to say, a classifier in which a particular type of information for input is taken as input and a type of information that is to be acquired is taken as output is configured using a machine learning method. For example, two or more pairs of information for input and output information are prepared in advance, the two or more pairs of information are given to a module for configuring a machine learning classifier to configure a classifier, and the configured classifier is accumulated in a storage unit. The classifier may also be said to be a learning model. There is no limitation on the machine learning method, and examples thereof include deep learning, random forests, and SVR. For example, functions in various machine learning frameworks and various existing libraries, such as fastText, tinySVM, random forest, and TensorFlow, can be used for the machine learning.

Furthermore, the classifier is not limited to those obtained through machine learning. The classifier may be a table indicating the correspondence between an input vector based on information for input or the like and output information, for example. In this case, output information corresponding to a feature vector based on information for input may be acquired from the table, or a vector that approximates a feature vector based on the information for input may be generated using two or more input vectors in the table and parameters for weighting each input vector, and output information corresponding to the input vectors and parameters used for generation may be used to acquire final output information. The classifier may be a function or the like that represents the relationship between an input vector based on the information for input or the like and information for generating output information, for example. In this case, for example, information corresponding to a feature vector based on information for input may be obtained using a function, and the obtained information may be used to acquire output information.

The outputting information is a concept that encompasses display on a display screen, projection using a projector, printing by a printer, output of a sound, transmission to an external apparatus, accumulation in a recording medium, and delivery of a processing result to another processing apparatus or another program. Specifically, for example, this concept encompasses enabling information to be displayed on a webpage, transmission as an email or the like, and outputting information for printing.

The accepting information is a concept that encompasses accepting information input from an input device such as a keyboard, a mouse, or a touch panel, receiving information transmitted from another apparatus or the like via a wired or wireless communication line, and accepting information read from a recording medium such as an optical disk, a magnetic disk, or a semiconductor memory.

The updating various types of information stored in the information processing apparatus or the like is a concept that encompasses changing the stored information, adding new information to the stored information, and deleting the whole or part of the stored information.

### Embodiment

In this embodiment, the information processing apparatus acquires a gut score related to a gut condition using input information containing sound information regarding abdominal sounds of a user and learning information prepared in advance, and outputs the gut score. The input information may contain life information regarding a life state of the user, and the life information may contain excretion-related information regarding an excretion status of the user, eating-and-drinking information regarding eating and drinking by the user, and activity status information regarding an activity status of the user. Preferably, the information processing apparatus can be used via a portable information terminal equipped with a microphone for recording abdominal sounds and a monitor on which scores are displayed, but there is no limitation to this. Hereinafter, an information processing system 1 with this configuration will be described.

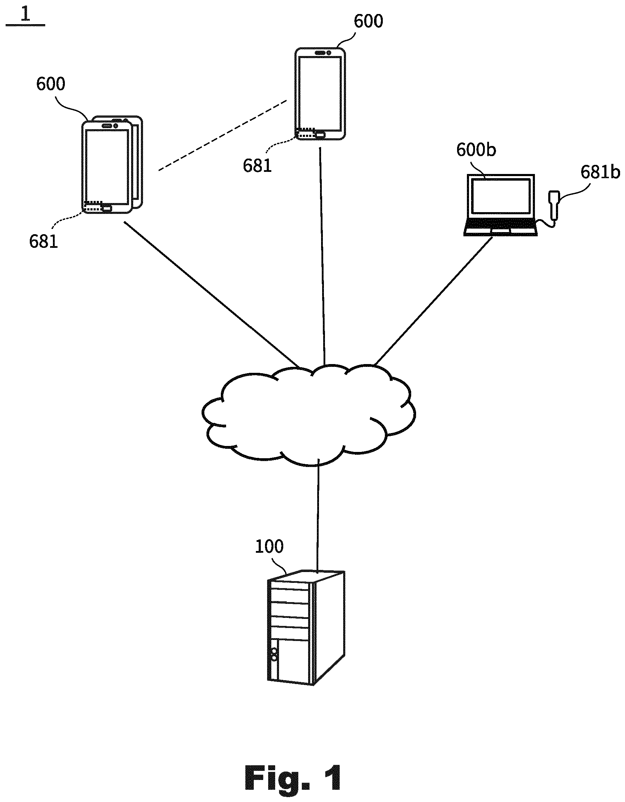

FIG. 1 is a diagram showing the outline of the information processing system 1 according to an embodiment of the present invention.

In this embodiment, the information processing system 1 includes an information processing apparatus 100 and terminal apparatuses 600. The information processing apparatus 100 and the terminal apparatuses 600 can communicate with each other via a network such as a local area network or the Internet, for example. The configuration of the information processing system 1 is not limited to this. There is no limitation on the number of each type of apparatuses included in the information processing system 1, and other apparatuses may be included in the information processing system 1.

Users of the information processing system 1 can use the information processing system 1 using the terminal apparatuses 600. In FIG. 1, portable information terminal apparatuses such as so-called smartphones, each of which has a built-in microphone 681, are shown as the terminal apparatuses 600, for example, but the terminal apparatuses 600 are not limited to such portable information terminal apparatuses. For example, terminal apparatuses 600b that are personal computers (PC) such as laptop computers may be used, or other apparatuses such as tablet-type information terminal apparatuses may be used. An external microphone 681b may be used as the microphone. In the following examples, it is assumed that portable information terminal apparatuses such as smartphones are used as the terminal apparatuses 600, but there is no limitation to this.

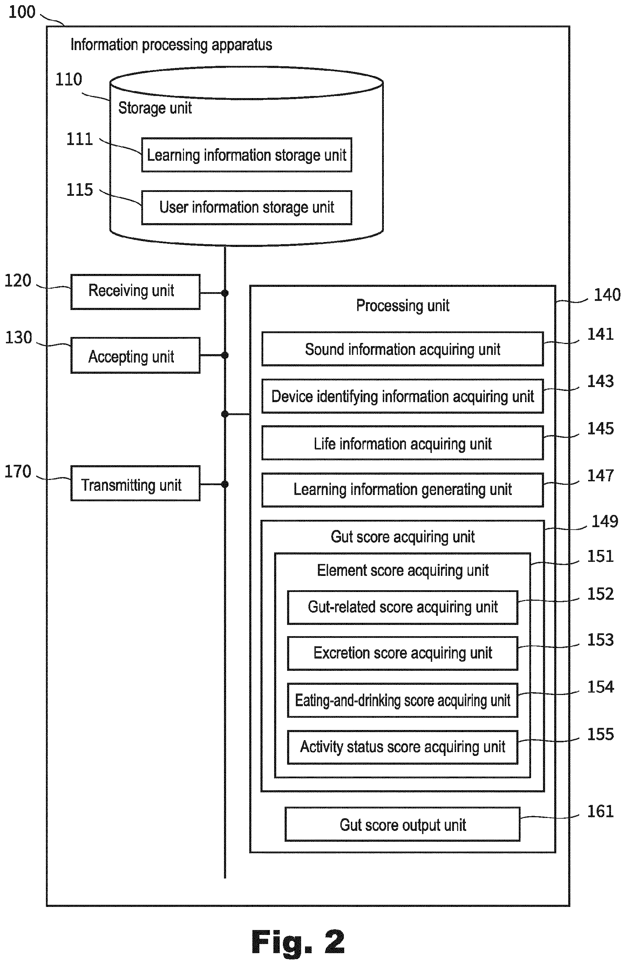

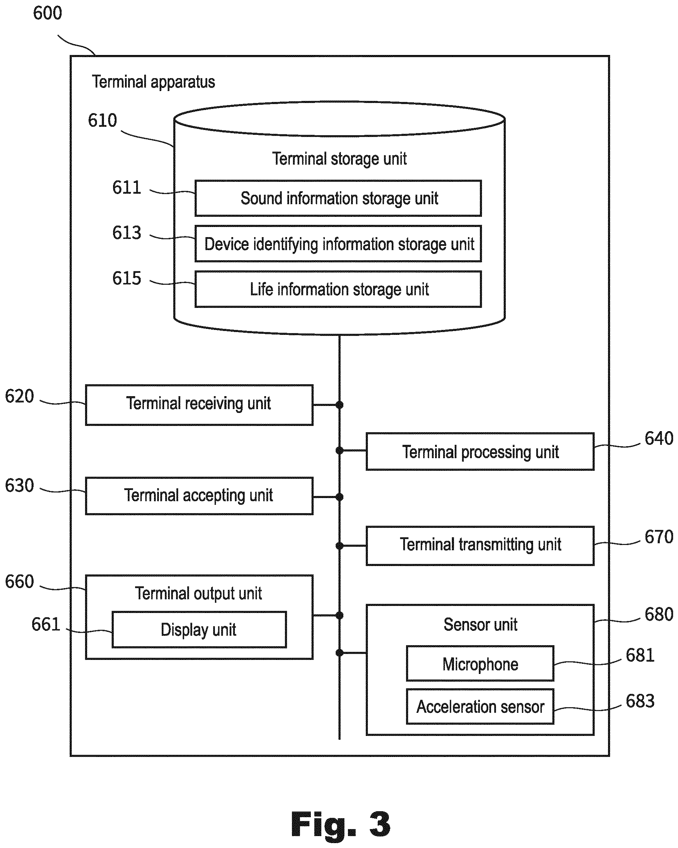

FIG. 2 is a block diagram of the information processing apparatus 100 in the embodiment. FIG. 3 is a block diagram of a terminal apparatus 600.

As shown in FIG. 2, the information processing apparatus 100 includes a storage unit 110, a receiving unit 120, an accepting unit 130, a processing unit 140, and a transmitting unit 170. The information processing apparatus 100 is a server apparatus, for example.

The storage unit 110 includes a learning information storage unit 111 and a user information storage unit 115.

Learning information acquired in advance is stored in the learning information storage unit 111. In this embodiment, the learning information is generated such that learning input information containing sound information is taken as information that is to be input and a value of a predetermined output indicator regarding an activity state of the guts is taken as information that is to be output. The learning information is generated using a so-called machine learning method. The learning information is generated by a learning information generating unit 147 and stored in the learning information storage unit 111, for example, but there is no limitation to this. That is to say, learning information generated by an apparatus that is different from the information processing apparatus 100 may be stored in the learning information storage unit 111.

In this embodiment, multiple pieces of learning information are prepared. The pieces of learning information are each stored in the learning information storage unit 111 in association with device identifying information. The device identifying information is information corresponding to the sound information contained in the learning input used to generate the learning information, and is information for identifying the type and the like of a device used to acquire, that is, record abdominal sounds corresponding to the sound information, for example. The device identifying information may also be said to be information for identifying a method used to record abdominal sounds, for example. The device may mean a microphone used to record abdominal sounds, or an apparatus set including the microphone, for example. The device identifying information may be, but is not limited to, a symbol for identifying the model of a device used to record abdominal sounds or a symbol for identifying the type of a device used to record abdominal sounds (e.g., whether a smartphone's built-in microphone was used or an external microphone was used), for example. Any symbol with which the origin of the learning input information, such as the recording method or the processing method of recorded abdominal sounds (e.g., whether or not a filter was applied, type, sound quality adjustment, etc.), can be identified at some granularity can be used as the device identifying information.

User information is stored in the user information storage unit 115. In this embodiment, the user information is information for associating a user identifier, which is an identifier for identifying a user who uses the information processing system 1, and information regarding the user. The user information may contain various types of information. For example, the user information may contain information transmitted from the terminal apparatus 600 that is used by the user, information on the user acquired by the information processing apparatus 100 as described later, and the like. The information transmitted from the terminal apparatus 600 that is used by the user corresponds to sound information, device identifying information, life information, and the like as described later, as well as the user's basic information, for example. The information on the user acquired by the information processing apparatus 100 corresponds to information regarding the frequency of gut sounds, a gut state estimation result, element scores, a gut score, and the like as described later, for example. User information transmitted from other external apparatuses and the like may be stored in the user information storage unit 115.

The receiving unit 120 receives information transmitted from another apparatus. The receiving unit 120 accumulates the received information in the storage unit 110, for example. In this embodiment, the user inputs information using the terminal apparatus 600 and transmits the information to the information processing apparatus 100, for example. The receiving unit 120 can accumulate each piece of transmitted information in the storage unit 110 in association with a user identifier. In this embodiment, it is also possible to receive sound information transmitted from each terminal apparatus 600 and accumulate the sound information in the storage unit 110 in association with a user identifier, as will be described later. In the case of receiving these pieces of information from the terminal apparatus 600, the receiving unit 120 can specify a user identifier of the user pertaining to the transmission based on the transmitted information.

The accepting unit 130 accepts information input using an input part (not shown) connected to the information processing apparatus 100. The accepting unit 130 accumulates the accepted information in the storage unit 110, for example. The information may be input by any part such as a numeric keypad, a keyboard, a mouse, or a menu screen. The accepting unit 130 may accept information input through an input operation performed using a reading apparatus (e.g., a code reader, etc.) connected to the information processing apparatus 100 (e.g., including information read by the apparatus).

The accepting unit 130 may be taken to accept the information received by the receiving unit 120, as information input to the information processing apparatus 100. That is to say, the input of information to the information processing apparatus 100 may mean that these pieces of information are indirectly input to the information processing apparatus 100 by the user via the terminal apparatus 600 or the like, or directly input to the information processing apparatus 100 by the user using an input part. The input of information to the information processing apparatus 100 may also be taken to mean that the information is given to the information processing apparatus 100 by the user executing a program that automatically generates information or giving various types of information to a program and causing the program to function.

The processing unit 140 includes a sound information acquiring unit 141, a device identifying information acquiring unit 143, a life information acquiring unit 145, a learning information generating unit 147, a gut score acquiring unit 149, an element score acquiring unit 151, and a gut score output unit 161. The processing unit 140 performs various types of processing. The various types of processing are processing that is performed by the units of the processing unit 140 as follows, for example.

The sound information acquiring unit 141 acquires sound information on a user. In this embodiment, the sound information acquiring unit 141 acquires sound information transmitted from the terminal apparatus 600 of the user and received by the receiving unit 120.

The device identifying information acquiring unit 143 acquires device identifying information corresponding to the sound information. In this embodiment, the device identifying information is, but is not limited to, information transmitted from the terminal apparatus 600 of the user in association with sound information. The device identifying information acquiring unit 143 may be configured to acquire device identifying information stored in the storage unit 110 as information for identifying the terminal apparatus 600 of the user in advance.

The life information acquiring unit 145 acquires life information. In this embodiment, the life information includes, but is not limited to, excretion-related information, eating-and-drinking information, and activity status information (i.e., exercise information or sleep information). The life information acquiring unit 145 acquires the information stored in the user information storage unit 115, for example. Furthermore, the life information acquiring unit 145 may acquire the user's basic information.

the learning information generating unit 147 generates learning information using a machine learning method. A machine learning method can be used as follows, for example. That is to say, a classifier in which learning input information containing sound information is taken as input and a value (output value) of a predetermined output indicator regarding an activity state of the guts is taken as output is configured using a machine learning method. For example, two or more pairs of learning input information and an output value are prepared in advance, the two or more pairs of information are given to a module for configuring a machine learning classifier to configure a classifier, and the configured classifier is accumulated in the learning information storage unit 111 as learning information. The classifier may also be said to be a learning model. There is no limitation on the machine learning method, and examples thereof include deep learning such as convolutional neural networks (CNN), random forests, and SVR. For example, functions in various machine learning frameworks and various existing libraries, such as fastText, tinySVM, random forest, and TensorFlow, can be used for the machine learning. It is sufficient that combinations of learning input information and an output value are prepared in advance. It is also possible to re-generate learning information at a predetermined point in time using a combination of sound information and an output value newly acquired by the information processing apparatus.

The gut score acquiring unit 149 acquires a gut score related to a gut condition of the user, using input information containing the sound information acquired by the sound information acquiring unit 141 and learning information prepared in advance. In this embodiment, the gut score acquiring unit 149 includes an element score acquiring unit 151. The element score acquiring unit 151 includes a gut-related score acquiring unit 152, an excretion score acquiring unit 153, an eating-and-drinking score acquiring unit 154, and an activity status score acquiring unit 155.

The gut score acquiring unit 149 in this embodiment acquires a value of a predetermined output indicator using the learning information stored in the learning information storage unit 111, and acquires a gut score using the value of the output indicator. The processing for acquiring a value of a predetermined output indicator is performed by the gut-related score acquiring unit 152, which will be described later, for example. For example, at least one of the bowel movement state and the number of peristalsis movements of the guts per unit time is used as the predetermined output indicator. The number of peristalsis movements of the guts may also be said to be the frequency of gut sounds at a predetermined level or greater (alternatively referred to simply as the "frequency of gut sounds" hereinafter). The gut score acquiring unit 149 is configured to acquire learning information corresponding to the device identifying information acquired by the device identifying information acquiring unit 143, from the learning information storage unit 111, and acquire a gut score using the acquired learning information.

In this embodiment, the gut score acquiring unit 149 acquires a gut score using the element scores acquired by the element score acquiring unit 151. The element score acquiring unit 151 causes the units to acquire element scores respectively for two or more evaluation elements based on the input information, as will be described later. The two or more evaluation elements may be gut-related elements, such as excretion, drink, food, an activity status, and the like, for example. That is to say, in this embodiment, for example, a gut movement score, a gut state score, an excretion score, a drink score, a food score, an activity status score, and the like are used as the element scores. The scores are symbols or the like indicating a numerical value, a grade, or the like representing the state or degree of goodness of a matter of interest, and is information easily understandable for users.

The gut-related score acquiring unit 152 acquires a gut state score regarding a bowel movement state (normal, diarrhea, constipation, etc.) and a gut movement score regarding peristalsis movements (frequency, etc.) of the guts, using the sound information acquired by the sound information acquiring unit 141 and learning information. In this embodiment, the gut-related score acquiring unit 152 inputs the sound information acquired by the sound information acquiring unit 141 to the learning information using a machine learning method, thereby acquiring an output indicator value. Then, the gut-related scores (a gut state score and a gut movement score) are acquired based on the output indicator value.

In this embodiment, the sound information that is used by the learning information generating unit 147 as learning input information and the sound information that is input by the gut-related score acquiring unit 152 are spectrum images obtained by representing, in a predetermined form, results of Fourier transform analysis or fast Fourier transform analysis performed on the sound data (which may be processed) obtained by recording abdominal sounds. The sound information may be sound data (which may be processed) itself or data converted to other formats. The sound information that is used by the learning information generating unit 147 as learning input information and the sound information that is input by the gut-related score acquiring unit 152 may be prepared by the processing unit 140. Sound information in the form of a spectrum image may be prepared in advance by a device other than the information processing apparatus 100, such as a device that recorded abdominal sounds, and transmitted to the information processing apparatus 100.

The learning information generating unit 147 and the gut-related score acquiring unit 152 may be configured to use learning input information containing user information such as basic information, in accordance with generation and use of the learning information. In this case, the learning information that is generated may be stored in the learning information storage unit 111, for each type by which users can be classified according to the user information, in association with an identifier for identifying the type. It is sufficient that the gut-related score acquiring unit 152 is configured to acquire learning information of a type corresponding to an identifier specified based on the user information, from the learning information storage unit 111, and acquire a gut-related score using the learning information. Accordingly, more accurate output results can be obtained.

The excretion score acquiring unit 153 acquires an excretion score based on the excretion-related information.

The eating-and-drinking score acquiring unit 154 acquires a drink score and a food score based on the eating-and-drinking information.

The activity status score acquiring unit 155 acquires an activity status score based on the activity status information.

These units of the element score acquiring unit 151 acquire element scores based on output indicator values or various types of life information, according to whether or not the content of the output indicator values or various types of life information satisfies a predetermined condition, for example. The predetermined condition may be set for each element and for each viewpoint of the element. For example, an element score can be acquired by reflecting a first predetermined point in the element score if a predetermined condition is satisfied or by reflecting a second predetermined point in the element score if the predetermined condition is not satisfied. Specifically, for example, in the case in which the frequency of gut sounds is obtained as an output indicator value, if the frequency of the gut sounds is within a predetermined range, a first predetermined point may be added to a gut movement score serving as the base, or otherwise a second predetermined point may be subtracted from the gut movement score serving as the base.

The units of the element score acquiring unit 151 accumulate each acquired element score in the user information storage unit 115 in association with a user identifier.

The units of the element score acquiring unit 151 may compare the content of output indicator values or various types of life information with a predetermined reference, and calculate an element score by using a predetermined calculation formula according to the comparison result. Also, multiple threshold values serving as a reference may be prepared, and a predetermined point corresponding to a condition range that the content of output indicator values or various types of life information matches may be reflected in the element score. Also, multiple evaluation viewpoints (viewpoints for comparison with a reference value, etc.) may be provided for each element such as an intestinal movement, a gut state, excretion, drink, food, or an activity status, and a predetermined point may be reflected in the element score according to a result of comparison with a reference value for each viewpoint. The corresponding point may be reflected in the element score based on information (e.g., an n-dimensional look-up table) that maps the points to be reflected, in advance, in a space composed of multiple viewpoint axes.

The units of the element score acquiring unit 151 may acquire an element score based on element scores acquired in the past for the user and stored in the user information storage unit 115, output indicator values or various types of life information in the past, or the like. For example, the units of the element score acquiring unit 151 may acquire a current element score by reflecting a current point in a previously acquired element score. It is also possible to acquire an element score using an average value or the like of output indicator values or various types of life information in a past predetermined period and current output indicator values or various types of life information. It is also possible to acquire an element score using points (by adding the points, etc.) respectively specified for a predetermined number of past output indicator values or various types of life information and current output indicator values or various types of life information.

The units of the element score acquiring unit 151 may set factors such as conditions set for acquiring such element scores, a reference value for use in comparison, an element score serving as the base, a point that is to be reflected in the element score, and a method for reflecting a point in the element score (addition, subtraction, multiplication, etc.) as appropriate based on the user's basic information. That is to say, the units of the element score acquiring unit 151 may acquire an element score based on the user's basic information. Specifically, for example, the units of the element score acquiring unit 151 may be configured to acquire an element score by applying different factors according to the user's sex and age.

In this embodiment, the gut score acquiring unit 149 acquires a gut score using the gut movement score and the gut state score acquired by the gut-related score acquiring unit 152 in this manner, the excretion score acquired by the excretion score acquiring unit 153, the drink score and the food score acquired by the eating-and-drinking score acquiring unit 154, and the activity status score acquired by the activity status score acquiring unit. The gut score may be acquired by applying element scores to a predetermined calculation formula and performing calculation such as addition or multiplication, for example. The gut score may be acquired by using a learning model configured using a machine learning method such that element scores are taken as input and a gut score is taken as output, for example. The element scores may be normalized or otherwise used to obtain a gut score, as necessary.

The gut score acquiring unit 149 accumulates the acquired gut score in the user information storage unit 115 in association with a user identifier.

The gut score acquiring unit 149 may acquire a current gut score based on gut scores acquired in the past for the user and stored in the user information storage unit 115. For example, the gut score acquiring unit 149 may acquire a current gut score by reflecting a point according to the current element scores in a score serving as the base based on the previous gut score.

The gut score acquiring unit 149 may change a predetermined method used to acquire such a gut score, as appropriate based on the user's basic information. Specifically, for example, the gut score acquiring unit 149 may be configured to acquire a gut score by performing calculation or the like using element scores according to different methods according to the user's sex, age, height, weight, medical history, results of predetermined questionnaires, and the like. That is to say, the gut score acquiring unit 149 may acquire a gut score based on the user's basic information.

The gut score output unit 161 outputs the gut score acquired by the gut score acquiring unit 149. In this embodiment, the gut score output unit 161 outputs information such as a gut score by transmitting it to the terminal apparatus 600, for example, but there is no limitation to this. For example, the output may be performed by displaying the gut score in text or images on a display screen included in the information processing apparatus 100.

In this embodiment, the gut score output unit 161 is configured to output a radar chart using the element scores acquired by the element score acquiring unit 151. In this case, the gut score output unit 161 may normalize element scores such that the element scores are balanced as necessary, and configure and output information representing the element scores in the form of a radar chart. Also, the gut score output unit 161 may configure and output information representing the element scores as a graph in other formats.

The transmitting unit 170 transmits information via a network to another apparatus constituting the information processing system 1. The transmitting unit 170 transmits information to the terminal apparatus 600, for example. In other words, the transmitting unit 170 outputs information to the terminal apparatus 600, for example.

Next, the configuration of the terminal apparatus 600 will be described.

As shown in FIG. 3, the terminal apparatus 600 includes a terminal storage unit 610, a terminal receiving unit 620, a terminal accepting unit 630, a terminal processing unit 640, a terminal output unit 660, a terminal transmitting unit 670, and a sensor unit 680. The terminal output unit 660 includes a display unit 661. The sensor unit 680 includes a microphone 681 and an acceleration sensor 683.

The terminal storage unit 610 includes a sound information storage unit 611, a device identifying information storage unit 613, and a life information storage unit 615.

Sound information recorded using the sensor unit 680 of the terminal apparatus 600 is accumulated in the sound information storage unit 611. The sound information, which is transmitted by the terminal transmitting unit 670 to the information processing apparatus 100, may be deleted from the sound information storage unit 611 when the transmission is completed, retained as it is until a predetermined period elapses, or retained permanently until a deletion operation is performed by the user.

Device identifying information is stored in the device identifying information storage unit 613. The device identifying information is, but is not limited to, information with which the model of the terminal apparatus 600 can be identified. Different information may not be writable to the device identifying information storage unit 613.

Life information is accumulated in the life information storage unit 615. The life information, which is transmitted by the terminal transmitting unit 670 to the information processing apparatus 100, may be deleted from the life information storage unit 615 when the transmission is completed, retained as it is until a predetermined period elapses, or retained permanently until a deletion operation is performed by the user.

The life information that is accumulated in the life information storage unit 615 may contain information input by the user or information based thereon. These pieces of information may be information accepted by the terminal accepting unit 630 or information acquired by the terminal processing unit 640 through calculation or the like based on the information accepted by the terminal accepting unit 630, for example.

Furthermore, the life information that is accumulated in the life information storage unit 615 may contain a measured value or information based thereon. These pieces of information may be information measured by the sensor unit 680 or information acquired by the terminal processing unit 640 through calculation or the like based on the information measured by the sensor unit 680, for example. Also, it may be information obtained through measurement or the like by a sensor apparatus communicably connected to the terminal apparatus 600 and transmitted to the terminal apparatus 600.

The terminal receiving unit 620 receives information transmitted from the information processing apparatus 100 or other apparatuses, via a network. The terminal receiving unit 620 accumulates the received information in the terminal storage unit 610, for example, such that it can be retrieved by the terminal processing unit 640 and the like.

The terminal accepting unit 630 accepts various operations input to the terminal apparatus 600 by the user who uses the terminal apparatus 600. The operations are performed using an input apparatus (not shown), for example, but there is no limitation to this. The terminal accepting unit 630 may accept an input operation by voice input using the microphone 681, for example.

The terminal processing unit 640 performs various information processing operations using the units of the terminal apparatus 600.

The terminal output unit 660 outputs information by displaying it on the display unit 661, which is a display device, for example. The method for outputting information is not limited to this, and may also be performed by outputting voice or the like from a speaker or the like.

The terminal transmitting unit 670 transmits information acquired by the terminal processing unit 640 or the like, via a network, for example.

The sensor unit 680 may include a barometric pressure sensor and the like, as well as the microphone 681 and the acceleration sensor 683, for example. The sensor unit 680 performs recording using the microphone 681 and measurement of measurement matters, and outputs information such as obtained sound data and measured value. The obtained information is accumulated in the terminal storage unit 610, for example. In this example, the measured value is, but is not limited to, a value indicating changes in acceleration acquired by the acceleration sensor, a value indicating changes in atmospheric pressure acquired by the barometric pressure sensor, or the like, for example. The sensor unit 680 may include a pulse sensor, an illuminance sensor, a camera, and a location information sensor that can identify the position by GPS or the like, for example. The sensor unit 680 may be a timer or the like that measures the passage of time. In this embodiment, the sensor unit 680 functions as an activity tracker for acquiring information regarding the user's level of activity such as the amount of calories burned and the number of steps and information regarding the user's lifestyle habits such as the user's wake-up time, bedtime, and commuting time. For example, information regarding the user's living environment such as the area in which the user lives, the climate, the noise environment, and the like may be acquired based on the measured values obtained by the sensor unit 680, and accumulated in the terminal storage unit 610.

Next, an example of an operation of the information processing apparatus 100 performed when a user uses the information processing system 1 according to this embodiment will be described. In this embodiment, the user can use the information processing system 1 by causing a predetermined application to work on the terminal apparatus 600 while accessing the information processing apparatus 100 via the terminal apparatus 600 or receiving information transmitted from the information processing apparatus 100, for example. The predetermined application may be a dedicated application that operates using information transmitted from the information processing apparatus 100, a web browser on which a web application provided by the information processing apparatus 100 is displayed in a usable manner, or the like, for example.

In this embodiment, the information processing system 1 is typically used as follows. That is to say, the user records his or her own abdominal sounds using the terminal apparatus 600. Then, sound information is transmitted from the terminal apparatus 600 to the information processing apparatus 100, and a gut score is acquired by the information processing apparatus 100. The information processing apparatus 100 transmits (outputs) the acquired gut score to the terminal apparatus 600. The terminal apparatus 600 receives the gut score, and the terminal output unit 660 displays information containing the gut score on a display device. Accordingly, the user can see information regarding his or her own guts as a gut score. In the case in which the information processing system 1 operates in this manner, the information processing apparatus 100 performs various operations as follows, for example. These operations are performed by the processing unit 140 executing control operations and the like while using the units.

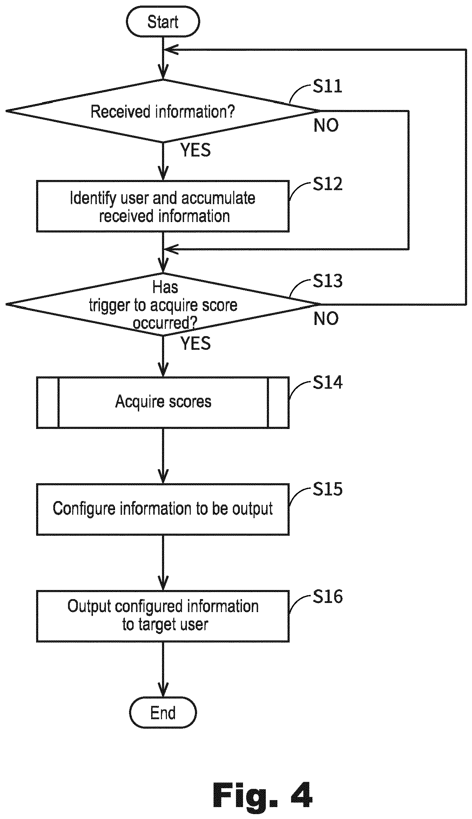

FIG. 4 is a flowchart showing an example of an operation of the information processing apparatus 100 in the embodiment.

(Step S11) The processing unit 140 determines whether or not information transmitted from the terminal apparatus 600 or the like has been received by the receiving unit 120. If it is determined that the information has been received, the procedure advances to step S12, or otherwise the procedure advances to step S13.

(Step S12) The processing unit 140 identifies the user based on the received information, and accumulates the received information in the user information storage unit 115 in association with a user identifier. For example, when life information, sound information, device identifying information, or the like is transmitted from the terminal apparatus 600 in association with a user identifier, the processing unit 140 accumulates the received information in the user information storage unit 115 in association with the user identifier.

(Step S13) The processing unit 140 determines whether or not a trigger to acquire a gut score has occurred. In other words, the processing unit 140 determines whether or not the conditions for starting acquisition of a gut score have been satisfied. If it is determined that the trigger has occurred, the procedure advances to step S14. Otherwise, the procedure returns to step S 11.

For example, the above-mentioned trigger may be a user's instruction to acquire a gut score through the terminal apparatus 600 (transmission of predetermined information corresponding to the instruction) or the like. The trigger is not limited to this. For example, the trigger may be fulfillment of various conditions such as arrival of a predetermined time or new receipt of sound information or other life information.

(Step S14) The processing unit 140 causes the gut score acquiring unit 149 to perform score acquiring processing. The score acquiring processing will be described later. Through the score acquiring processing, the gut score and the element scores are acquired and accumulated in the user information storage unit 115.

(Step S15) The processing unit 140 configures information that is to be output, using the gut score acquired by the gut score output unit 161 and the like. In this embodiment, information for showing the gut score and information for showing the element scores in the form of a radar chart are configured.

(Step S16) The processing unit 140 causes the gut score output unit 161 to output the configured information to the target user. That is to say, the processing unit 140 causes the transmitting unit 170 to output the information configured for the gut score and the element scores to the terminal apparatus 600 that is used by the user whose scores are to be calculated. Accordingly, the terminal apparatus 600 that received the information can perform display regarding the gut score and the element scores.

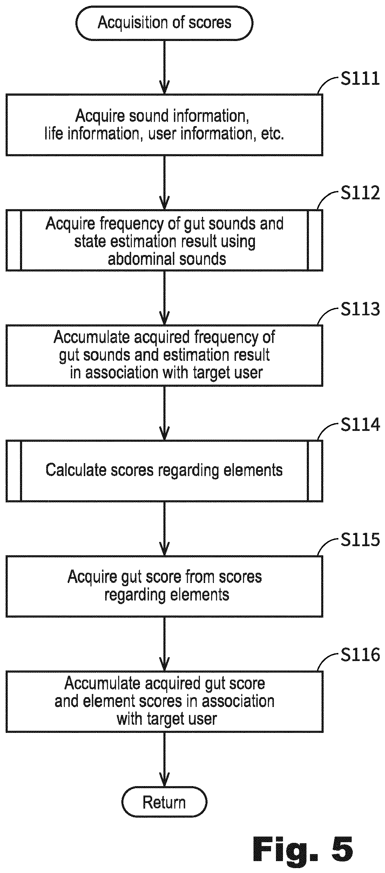

FIG. 5 is a flowchart showing an example of the score acquiring processing of the information processing apparatus 100 in the embodiment.

(Step Sill) The gut score acquiring unit 149 acquires sound information, life information, past user information, and the like regarding a target user from the user information storage unit 115 based on the user identifier of the user. The gut score acquiring unit 149 may acquire basic information.

(Step S112) The gut score acquiring unit 149 performs processing for acquiring the frequency of gut sounds and a gut state estimation result using the sound information. Hereinafter, this processing may be referred to as abdominal sound usage processing. The abdominal sound usage processing will be described later in detail.

(Step S113) The gut score acquiring unit 149 accumulates information such as the acquired frequency of gut sounds and gut state estimation result, in the user information storage unit 115 in association with the user identifier of the target user.

(Step S114) The gut score acquiring unit 149 causes the element score acquiring unit 151 to perform processing for acquiring a score regarding each element. Hereinafter, this processing may be referred to as element score acquiring processing. In this embodiment, gut-related score acquiring processing, excretion score acquiring processing, food score acquiring processing, drink score acquiring processing, and activity status score acquiring processing are performed as the element score acquiring processing. Each element score acquiring processing will be described later in detail.

(Step S115) When the element scores are obtained, the gut score acquiring unit 149 acquires a gut score from the element scores. At this time, the gut score acquiring unit 149 acquires a gut score using a predetermined method such as addition or multiplication of the element scores as described above, for example, but there is no limitation to this.

(Step S116) The gut score acquiring unit 149 accumulates the acquired gut score and element scores in the user information storage unit 115 in association with the user identifier of the target user. Subsequently, the procedure returns to the processing in FIG. 4.

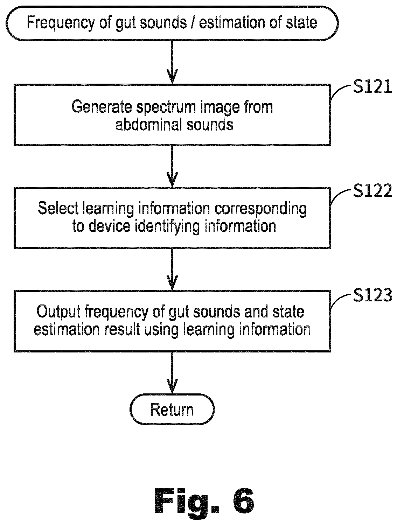

FIG. 6 is a flowchart showing an example of the abdominal sound usage processing of the information processing apparatus 100 in the embodiment.

(Step S121) The gut score acquiring unit 149 generates a spectrum image from sound information, which is sound data obtained by recording abdominal sounds.

(Step S122) The gut score acquiring unit 149 selects, as learning information that is to be used, learning information corresponding to the device identifying information corresponding to the sound information, out of the learning information stored in the learning information storage unit 111.

(Step S123) The gut score acquiring unit 149 inputs the sound information to the learning information, and outputs an output indicator value. In this embodiment, the frequency of gut sounds contained in the abdominal sounds and a gut state estimation result are output. Subsequently, the procedure returns to the processing shown in FIG. 5.

In a case in which the sound information stored in the user information storage unit 115 is a spectrum image such as a case in which the sound information transmitted from the terminal apparatus 600 is a spectrum image, the processing in step S121 may not be performed.

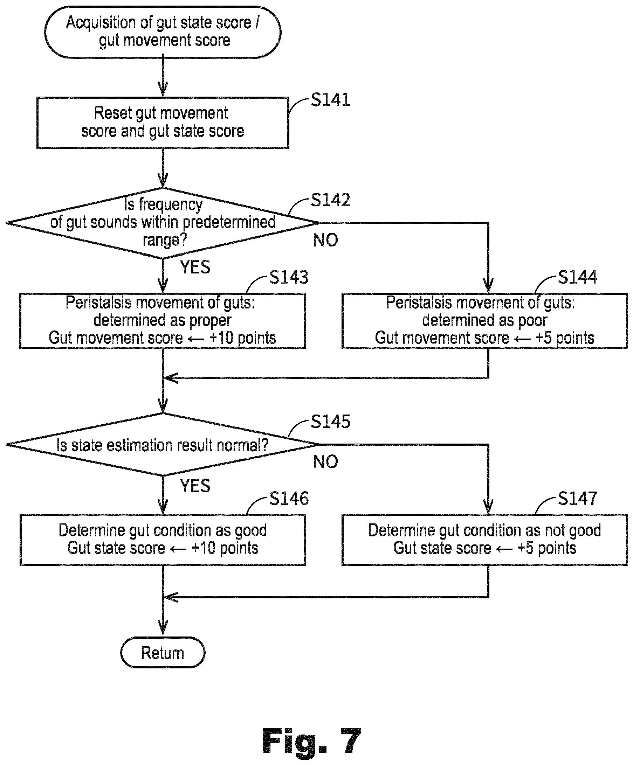

FIG. 7 is a flowchart showing an example of the gut-related score acquiring processing of the information processing apparatus 100 in the embodiment.

(Step S141) The gut-related score acquiring unit 152 resets the gut movement score and the gut state score respectively to initial values (e.g., zero).

(Step S142) The gut-related score acquiring unit 152 determines whether or not the frequency of gut sounds is within a predetermined range. If it is determined that the frequency is within the predetermined range, the procedure advances to step S143, or otherwise the procedure advances to step S144.

The state in which the frequency of gut sounds is within a predetermined range can be set to a state in which the number of gut sounds greater than a predetermined level during a predetermined length of time is 5 or more and less than 20, for example, but the number and the like are not limited to this.

(Step S143) The gut-related score acquiring unit 152 adds 10 points to the gut movement score. That is to say, the gut-related score acquiring unit 152 determines that the peristalsis movement of the guts is in a proper state. The procedure advances to step S145.

(Step S144) The gut-related score acquiring unit 152 adds 5 points to the gut movement score. That is to say, the gut-related score acquiring unit 152 determines that the peristalsis movement of the guts is in a poor state. The procedure advances to step S145.

(Step S145) The gut-related score acquiring unit 152 determines whether or not the gut state estimation result is "normal". If it is determined as being within a predetermined range, the procedure advances to step S143, or otherwise the procedure advances to step S144. If the gut state estimation result is "diarrhea" or "constipation", the procedure advances to step S147.

(Step S146) The gut-related score acquiring unit 152 adds 10 points to the gut state score. That is to say, the gut-related score acquiring unit 152 determines that the gut condition is in a good state.

(Step S147) The gut-related score acquiring unit 152 adds 5 points to the gut state score. That is to say, the gut-related score acquiring unit 152 determines that the peristalsis movement of the guts is in a proper state.

When step S146 or step S147 is ended, the gut-related score acquiring unit 152 acquires a gut movement score and a gut state score as a result of the above-described processing, and the procedure returns to FIG. 5.

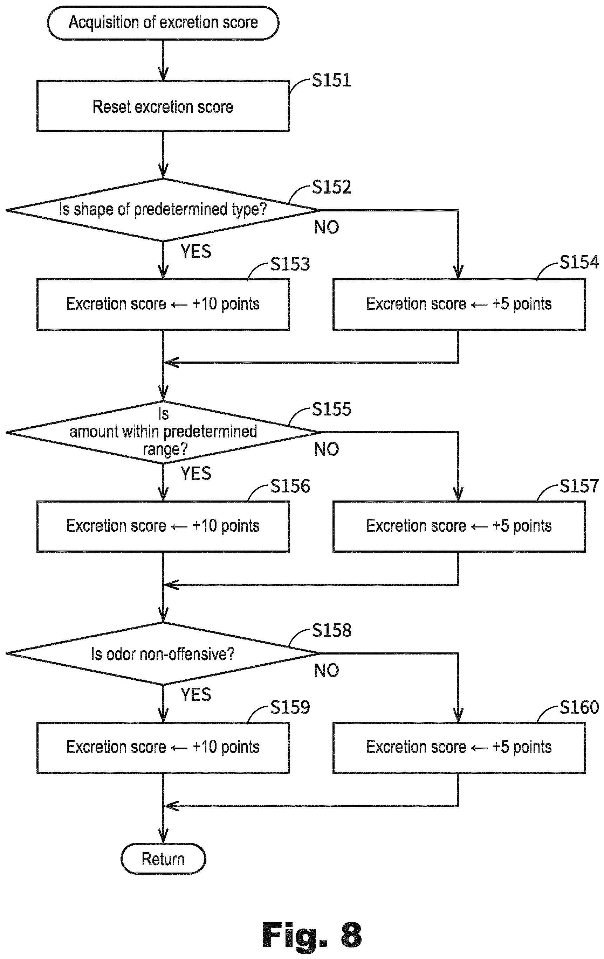

FIG. 8 is a flowchart showing an example of the excretion score acquiring processing of the information processing apparatus 100 in the embodiment.

(Step S151) The excretion score acquiring unit 153 resets the excretion score to an initial value (e.g., zero).

(Step S152) The excretion score acquiring unit 153 determines whether or not the shape of excrement corresponds to a predetermined type. For example, if the label value of the Bristol Scale contained in the excretion-related information is a predetermined value (e.g., a predetermined word such as "banana" or "sausage"), it can be determined that the shape of excrement corresponds to a predetermined type. If it is determined that the shape of excrement corresponds to the predetermined type, the procedure advances to step S153, or otherwise the procedure advances to step S154.

(Step S153) The excretion score acquiring unit 153 adds 10 points to the excretion score. The procedure advances to step S155.

(Step S154) The excretion score acquiring unit 153 adds 5 points to the excretion score. The procedure advances to step S155.

(Step S155) The excretion score acquiring unit 153 determines whether or not the amount of excrement is within a predetermined range. For example, the user's input value contained in the excretion-related information and indicating the amount of excrement can be used as the amount of excrement. For example, the predetermined range can be set to, but is not limited to, a range from 0 to 200 grams. If it is determined that the amount of excrement is within the predetermined range, the procedure advances to step S156, or otherwise the procedure advances to step S157.

(Step S156) The excretion score acquiring unit 153 adds 10 points to the excretion score. The procedure advances to step S158.

(Step S157) The excretion score acquiring unit 153 adds 5 points to the excretion score. The procedure advances to step S158.

(Step S158) The excretion score acquiring unit 153 determines whether or not the odor of excrement is non-offensive. For example, the user's input value contained in the excretion-related information and indicating the odor of excrement can be used as the odor of excrement. If it is determined that the odor of excrement is non-offensive, the procedure advances to step S159, or otherwise the procedure advances to step S160.

(Step S159) The excretion score acquiring unit 153 adds 10 points to the excretion score.

(Step S160) The excretion score acquiring unit 153 adds 5 points to the excretion score.

When step S159 or step S160 is ended, the excretion score acquiring unit 153 acquires an excretion score as a result of the above-described processing, and the procedure returns to FIG. 5.

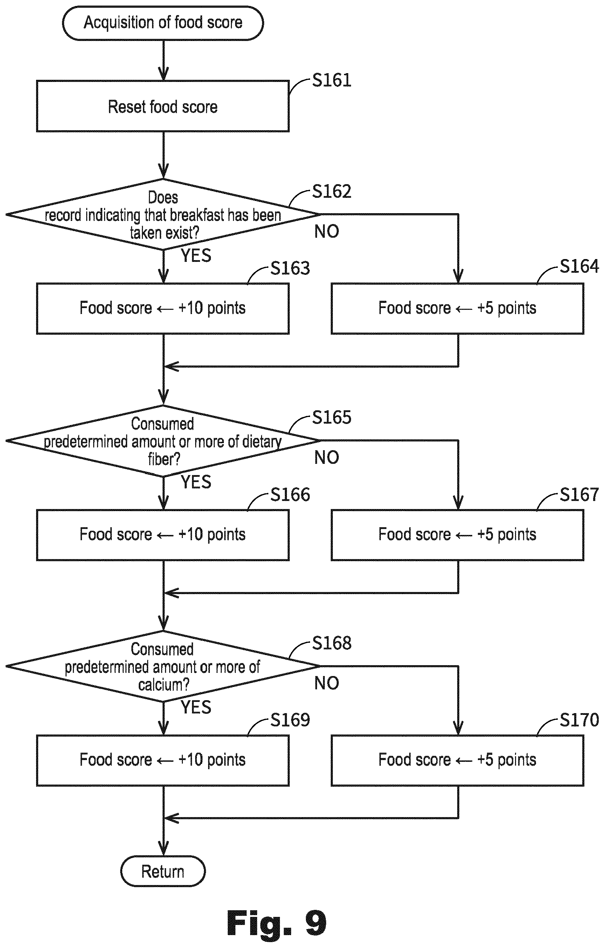

FIG. 9 is a flowchart showing an example of the food score acquiring processing of the information processing apparatus 100 in the embodiment.

(Step S161) The eating-and-drinking score acquiring unit 154 resets the food score to an initial value (e.g., zero).

(Step S162) The eating-and-drinking score acquiring unit 154 determines whether or not the user has taken the breakfast. For example, this determination can be made based on whether or not there is an input value contained in the food information and indicating that the user has taken the breakfast, or the like. If it is determined that the user has taken the breakfast, the procedure advances to step S163, or otherwise the procedure advances to step S164.

(Step S163) The eating-and-drinking score acquiring unit 154 adds 10 points to the food score. The procedure advances to step S165.

(Step S164) The eating-and-drinking score acquiring unit 154 adds 5 points to the food score. The procedure advances to step S165.

(Step S165) The eating-and-drinking score acquiring unit 154 determines whether or not the user has consumed a predetermined amount or more of dietary fiber. For example, this determination can be made using a value obtained by calculating the dietary fiber equivalent based on information contained in the food information and indicating the content of a meal. For example, the predetermined amount can be set to, but is not limited to, 12 grams. An upper limit may be set. If it is determined that the user has consumed the predetermined amount or more of dietary fiber, the procedure advances to step S166, or otherwise the procedure advances to step S167.

(Step S166) The eating-and-drinking score acquiring unit 154 adds 10 points to the food score. The procedure advances to step S168.

(Step S167) The eating-and-drinking score acquiring unit 154 adds 5 points to the food score. The procedure advances to step S168.

(Step S168) The eating-and-drinking score acquiring unit 154 determines whether or not the user has consumed a predetermined amount or more of calcium. For example, this determination can be made using a value obtained by calculating the calcium equivalent based on information contained in the food information and indicating the content of a meal. For example, the predetermined amount can be set to, but is not limited to, 160 milligrams. An upper limit may be set. If it is determined that the user has consumed the predetermined amount or more of calcium, the procedure advances to step S169, or otherwise the procedure advances to step S160.

(Step S169) The eating-and-drinking score acquiring unit 154 adds 10 points to the food score.

(Step S170) The eating-and-drinking score acquiring unit 154 adds 5 points to the food score.

When step S169 or step S170 is ended, the eating-and-drinking score acquiring unit 154 acquires a food score as a result of the above-described processing, and the procedure returns to FIG. 5.

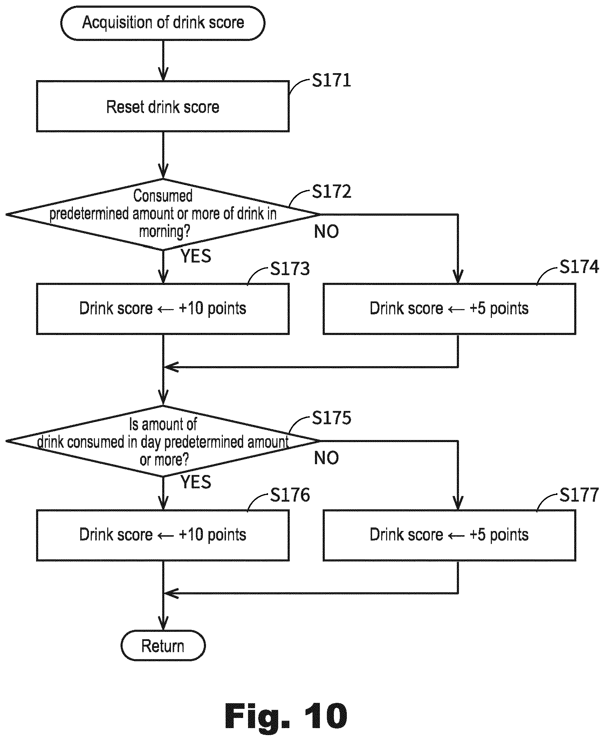

FIG. 10 is a flowchart showing an example of the drink score acquiring processing of the information processing apparatus 100 in the embodiment.

(Step S171) The eating-and-drinking score acquiring unit 154 resets the drink score to an initial value (e.g., zero).

(Step S172) The eating-and-drinking score acquiring unit 154 determines whether or not the user has consumed a predetermined amount or more of drink in the morning. For example, this determination can be made based on an input value contained in the drink information and indicating the amount of water consumed by the user during a predetermined timeframe or the like. For example, the predetermined amount can be set to, but is not limited to, 300 milliliters. An upper limit may be set. If it is determined that the user has consumed the predetermined amount or more of drink in the morning, the procedure advances to step S173, or otherwise the procedure advances to step S174.

(Step S173) The eating-and-drinking score acquiring unit 154 adds 10 points to the drink score. The procedure advances to step S175.

(Step S174) The eating-and-drinking score acquiring unit 154 adds 5 points to the drink score. The procedure advances to step S175.

(Step S175) The eating-and-drinking score acquiring unit 154 determines whether or not the amount of drink consumed by the user in a day is a predetermined amount or more. For example, this determination can be made based on an input value contained in the drink information and indicating the amount of water consumed by the user in a day or the like. For example, the predetermined amount can be set to, but is not limited to, 2000 milliliters. An upper limit may be set. If it is determined that the amount of drink consumed in a day is the predetermined amount or more, the procedure advances to step S176, or otherwise the procedure advances to step S177.

(Step S176) The eating-and-drinking score acquiring unit 154 adds 10 points to the drink score.

(Step S177) The eating-and-drinking score acquiring unit 154 adds 5 points to the drink score.

When step S176 or step S177 is ended, the eating-and-drinking score acquiring unit 154 acquires a drink score as a result of the above-described processing, and the procedure returns to FIG. 5.

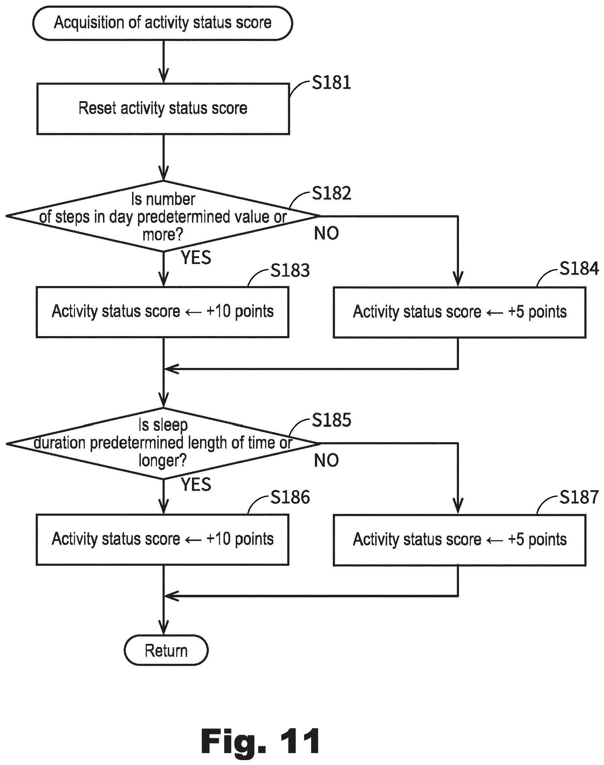

FIG. 11 is a flowchart showing an example of the activity status score acquiring processing of the information processing apparatus 100 in the embodiment.

(Step S181) The activity status score acquiring unit 155 resets the activity status score to an initial value (e.g., zero).

(Step S182) The activity status score acquiring unit 155 determines whether or not the number of steps in a day is a predetermined value or more. For example, this determination can be made based on an accumulated value contained in the activity status information and indicating the number of steps taken by the user in a day or the like. For example, the predetermined value can be set to, but is not limited to, 7000 steps. An upper limit may be set. If it is determined that the number of steps in a day is the predetermined value or more, the procedure advances to step S183, or otherwise the procedure advances to step S184.

(Step S183) The activity status score acquiring unit 155 adds 10 points to the activity status score. The procedure advances to step S185.

(Step S184) The activity status score acquiring unit 155 adds 5 points to the activity status score. The procedure advances to step S185.

(Step S185) The activity status score acquiring unit 155 determines whether or not the user's sleep duration is a predetermined length of time or longer. For example, this determination can be made based on an input value contained in the activity status information and indicating the user's sleep duration. For example, the predetermined length of time can be set to, but is not limited to, 7 hours. An upper limit may be set. If it is determined that the sleep duration is the predetermined length of time or longer, the procedure advances to step S186, or otherwise the procedure advances to step S187.

(Step S186) The activity status score acquiring unit 155 adds 10 points to the activity status score.

(Step S187) The activity status score acquiring unit 155 adds 5 points to the activity status score.

When step S186 or step S187 is ended, the activity status score acquiring unit 155 acquires an activity status score as a result of the above-described processing, and the procedure returns to FIG. 5.

Next, specific examples of an operation of the information processing system 1 in this embodiment will be described with reference to the screen transition of the terminal apparatus 600 that is used by the user.

In the following specific examples, a case is assumed in which the information processing system 1 provides a health support application to assist a user to lead a healthy life. The health support application accepts input operations related to life information of a user and provides the user with information useful for maintaining good health. The health support application is realized when the user executes a predetermined application on the terminal apparatus 600 and communication is performed between the terminal apparatus 600 and the information processing apparatus 100. The following screen examples of the health support application are displayed by the terminal output unit 660 based on the control by the terminal processing unit 640.

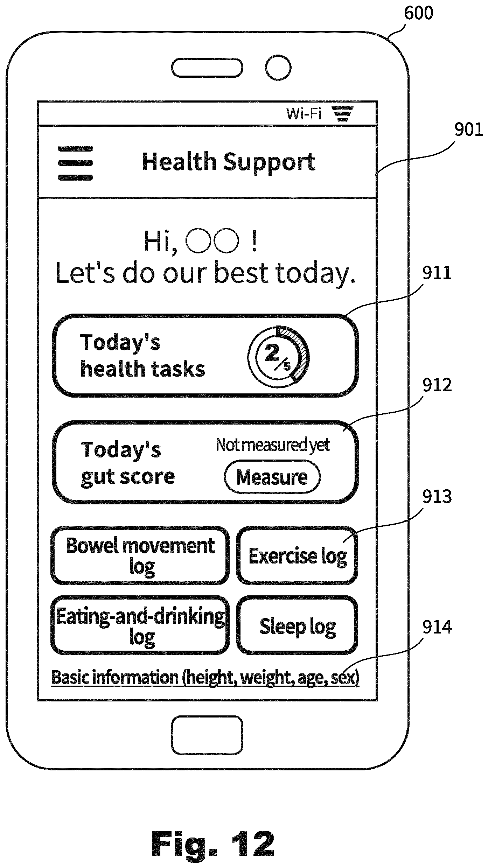

FIG. 12 is a first diagram showing a specific example of screen transition of the terminal apparatus 600.

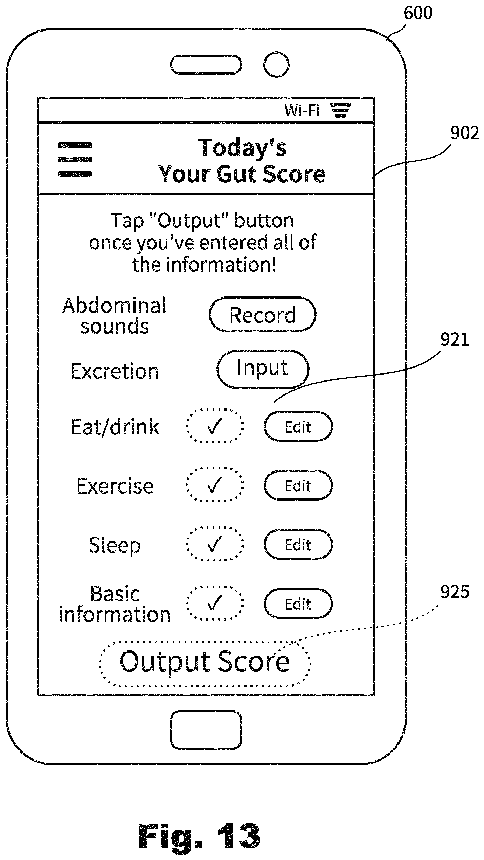

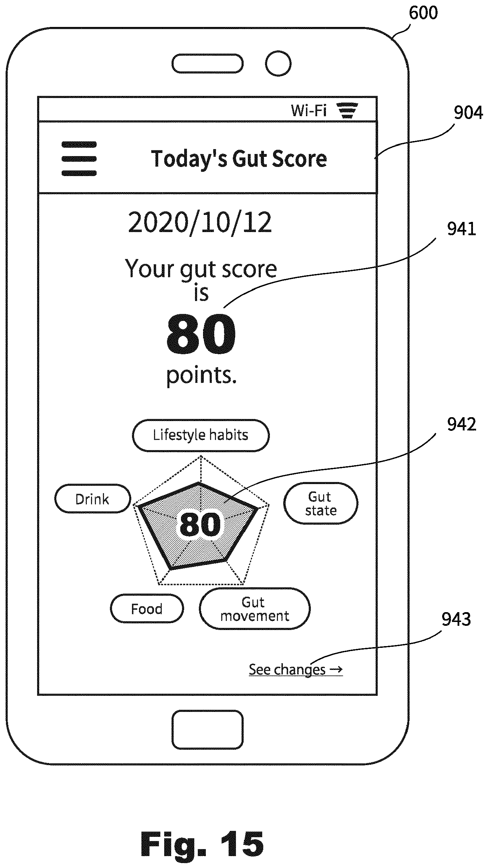

FIG. 12 shows a menu screen 901 of a health support application. The menu screen 901 includes a task display unit 911 that displays information on tasks (health tasks) recommended to be performed by the user, a gut score information display unit 912 that displays information regarding a gut score, life information record buttons 913 for performing input regarding life information and viewing history, a basic information button 914 for checking and editing basic information, and the like, for example. The task display unit 911 includes an indication of the progress of a task, for example. When the task display unit 911 is operated, the screen transitions to a task management screen (not shown). In this specific example, if no gut score has been obtained within a past predetermined period, the gut score information display unit 912 includes an indication to that effect, for example. When the gut score information display unit 912 is operated, the screen transitions to a score acquisition instruction screen 902 (shown in FIG. 13) for output of a gut score, for example. On the other hand, if a gut score has been obtained within a past predetermined period, the gut score information display unit 912 includes an indication of a measurement result of the gut score, for example. When the gut score information display unit 912 is operated, the screen transitions to a score display screen 904 (shown in FIG. 15) including an indication of a measurement result of the gut score, for example. The user can operate the life information record buttons 913 to input and record information regarding each element and to transmit the information to the information processing apparatus 100 for storage in the information processing apparatus 100.

FIG. 13 is a second diagram showing a specific example of screen transition of the terminal apparatus 600 in the embodiment.

FIG. 13 shows the score acquisition instruction screen 902 of the health support application. The score acquisition instruction screen 902 is a screen for outputting a gut score to the information processing apparatus 100. That is to say, the score acquisition instruction screen 902 includes a confirmation area 921 that indicates whether or not all pieces of information necessary for outputting a gut score are available, and an instruction button 925 for instructing the output of the gut score. The confirmation area 921 includes an indication of whether or not information such as life information and abdominal sounds used to output a gut score has already been prepared (input) in the information processing apparatus 100 or the terminal apparatus 600. For information that is missing, a button for preparing that information is displayed, but there is no limitation to this. The instruction button 925 is disabled until life information and abdominal sounds used to output a gut score are all available, and is enabled (or may be said to appear) when these pieces of information are all available, for example. When the instruction button 925 is operated, the information processing apparatus 100 acquires a gut score as described above and transmits the acquired gut score and the like to the terminal apparatus 600. When the terminal apparatus 600 receives the gut score and the like, the screen transitions to the score display screen 904 (shown in FIG. 15).

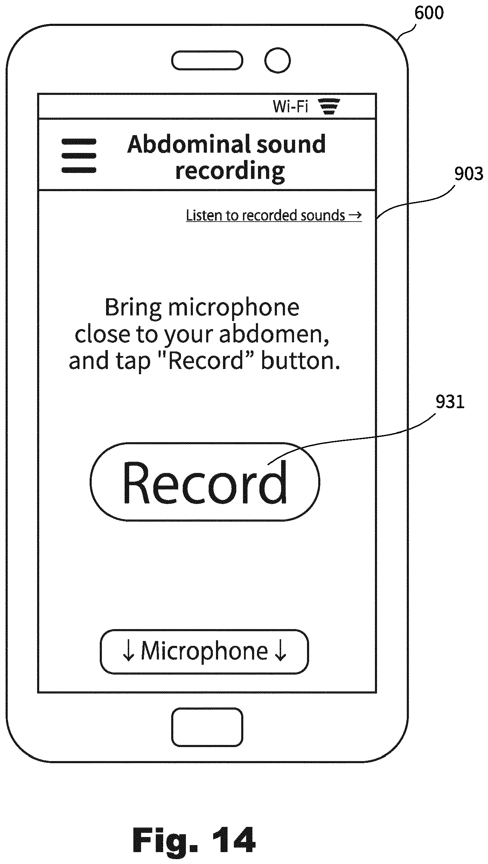

In FIG. 13, information is missing for abdominal sounds and excretion. When a button displayed corresponding to abdominal sounds ("Record" button) is operated, the screen transitions to an abdominal sound record screen 903 (shown in FIG. 14).

FIG. 14 is a third diagram showing a specific example of screen transition of the terminal apparatus 600 in the embodiment.

FIG. 14 shows the abdominal sound record screen 903 of the health support application. The abdominal sound record screen 903 includes a record button 931 for starting recording. When the record button 931 is operated, recording is performed using the microphone 681, and sound information is stored in the sound information storage unit 611. The abdominal sound record screen 903 may include an indication of the location of the microphone 681. A guide display or the like of an appropriate recording method regarding the posture of the user and the posture and position of the terminal apparatus 600 may also be displayed on the abdominal sound record screen 903. With such indications, the user can easily and appropriately record abdominal sounds by bringing the microphone 681 close to the abdomen.

FIG. 15 is a fourth diagram showing a specific example of screen transition of the terminal apparatus 600 in the embodiment.

FIG. 15 shows the score display screen 904 of the health support application. The score display screen 904 includes a gut score display unit 941 that shows an acquired gut score and an element score display unit 942 that shows element scores in the form of a radar chart, for example. The user can check the gut score and the element scores on the score display screen 904. Since the element scores are shown in the form of a radar chart, the user can intuitively know which elements may be lacking in efforts with respect to maintaining a high gut condition. In the example shown in FIG. 15, a history display button 943 is included to check the changes including past scores as well. The user can operate the history display button 943 to check changes in the gut score and the element scores.

As explained above, it can be said that the information processing apparatus 100 can realize the following information processing method using the learning information and the sound information stored in the storage unit 110. That is to say, the information processing apparatus 100 acquires sound information regarding abdominal sounds, which are sounds emanating from the abdomen, of a user, acquires a gut score related to a gut condition of the user, using input information containing the acquired sound information and learning information prepared in advance, and outputs the acquired gut score. Since the gut score acquired based on the sound information is output, the user can easily obtain objective and easily understandable information on his or her own gut condition.

In this embodiment, a gut score is acquired using life information on elements that are thought to be related to the gut condition. Accordingly, it is possible to output a more accurate gut score. It is possible to output a more accurate gut score also in the case in which the gut score is acquired using the user's basic information. The gut score is acquired using information regarding multiple elements. Accordingly, it is possible to output an even more accurate gut score. In this embodiment, multiple element scores are acquired, and a gut score is acquired using these scores. Accordingly, the user can easily know which elements may be lacking in efforts with respect to maintaining a high gut condition and which elements may be a contributing factor to poor gut conditions. The multiple element scores are output in the form of a graph such as a radar chart. Accordingly, the user can intuitively see evaluation results for each element related to the gut condition.

### Description of Modified Examples

Hereinafter, modified examples of the foregoing embodiment will be described.

A first modified example is as follows. That is to say, in the abdominal sound usage processing, the extraction of the frequency of gut sounds and the estimation of the gut state may be performed using learning information configured using mutually different pieces of input information. The element scores may be acquired not only through addition of evaluation points to the element score but also subtraction and the like.

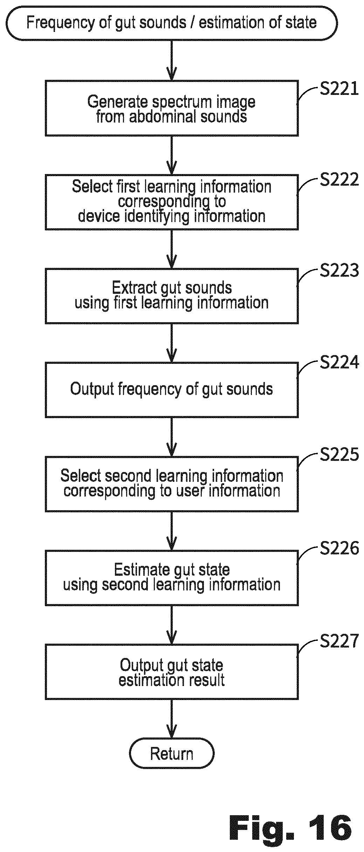

FIG. 16 is a flowchart showing an example of the abdominal sound usage processing of the information processing apparatus 100 according to a first modified example of the embodiment of the present invention.

(Step S221) The gut score acquiring unit 149 generates a spectrum image from sound information, which is sound data obtained by recording abdominal sounds.

(Step S222) The gut score acquiring unit 149 selects, as learning information that is to be used, first learning information corresponding to the device identifying information corresponding to the sound information, out of the learning information stored in the learning information storage unit 111. The first learning information is learning information configured using a machine learning method such that sound information is taken as input and sound information of the gut sound portion is taken as output, for example.

(Step S223) The gut score acquiring unit 149 inputs the sound information to the first learning information using the machine learning method, thereby extracting gut sounds.

(Step S224) The gut score acquiring unit 149 outputs the frequency of gut sounds based on information on the extracted gut sounds.

(Step S225) The gut score acquiring unit 149 selects, as learning information that is to be used, second learning information corresponding to the user information, out of the learning information stored in the learning information storage unit 111. The second learning information is learning information configured using a machine learning method such that predetermined life information (e.g., excretion information, eating-and-drinking information, etc.) as well as the sound information is taken as input and a gut state estimation result is taken as output, for example.

(Step S226) The gut score acquiring unit 149 inputs the sound information and the predetermined life information to the second learning information using the machine learning method, thereby acquiring a gut state estimation result.

(Step S227) The gut score acquiring unit 149 outputs information on the gut state estimation result. Subsequently, the procedure returns to the processing shown in FIG. 5.

If the abdominal sound usage processing is performed in this manner, the frequency of gut sounds and the gut state estimation result can be obtained with greater accuracy.

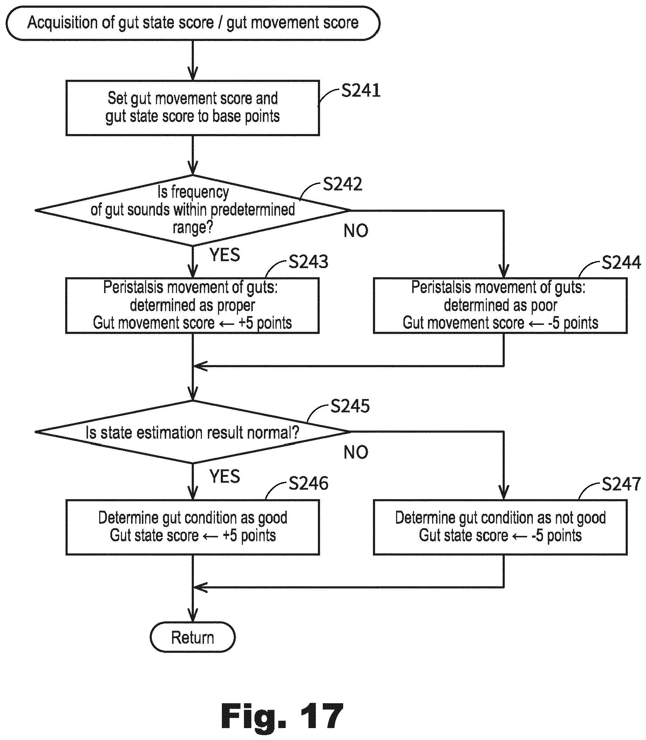

FIG. 17 is a flowchart showing an example of the gut-related score acquiring processing of the information processing apparatus 100 in the embodiment.

(Step S241) The gut-related score acquiring unit 152 sets the gut movement score and the gut state score respectively to base points (e.g., 10 points).

(Step S242) The gut-related score acquiring unit 152 determines whether or not the frequency of gut sounds is within a predetermined range. If it is determined that the frequency is within the predetermined range, the procedure advances to step S243, or otherwise the procedure advances to step S244.

(Step S243) The gut-related score acquiring unit 152 adds 5 points to the gut movement score. That is to say, the gut-related score acquiring unit 152 determines that the peristalsis movement of the guts is in a proper state. The procedure advances to step S245.

(Step S244) The gut-related score acquiring unit 152 subtracts 5 points from the gut movement score. That is to say, the gut-related score acquiring unit 152 determines that the peristalsis movement of the guts is in a poor state. The procedure advances to step S245.

(Step S245) The gut-related score acquiring unit 152 determines whether or not the gut state estimation result is "normal". If it is determined as being within a predetermined range, the procedure advances to step S243, or otherwise the procedure advances to step S244.

(Step S246) The gut-related score acquiring unit 152 adds 5 points to the gut state score. That is to say, the gut-related score acquiring unit 152 determines that the gut condition is in a good state.

(Step S247) The gut-related score acquiring unit 152 subtracts 5 points from the gut state score. That is to say, the gut-related score acquiring unit 152 determines that the peristalsis movement of the guts is in a proper state.

Such a method can be employed in obtaining other element scores as well. If this method is used, for each element score, a determination result for the reference and the degree to which element score is affected thereby can be easily adjusted.

The base point of the element scores may be a predetermined point or may be set based on past history or other factors. For example, a previously acquired element score may be set as the current base point. Alternatively, a value (e.g., an average value, etc.) obtained through calculation based on information on past element scores in a predetermined period may be set as the base point. It will be appreciated that the same applies to the foregoing embodiment.

A second modified example is as follows. In the abdominal sound usage processing, learning information that is to be used may be selected based on life information. In this case, the learning information stored in the learning information storage unit 111 may be associated with one or more elements and one or more viewpoints that may be contained in the life information, and, in the abdominal sound usage processing, learning information that is to be used may be selected based on the one or more elements and the one or more viewpoints in the life information.

For example, the gut score acquiring unit 149 may select learning information that is to be used in the abdominal sound usage processing, based on the acquired excretion-related information.

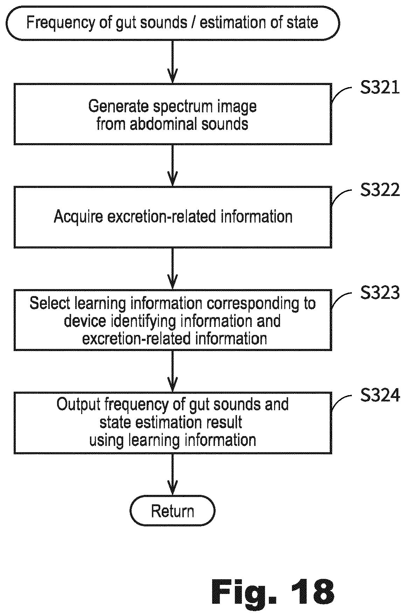

FIG. 18 is a flowchart showing an example of the abdominal sound usage processing of the information processing apparatus 100 according to a second modified example of the embodiment of the present invention.

(Step S321) The gut score acquiring unit 149 generates a spectrum image from sound information, which is sound data obtained by recording abdominal sounds.

(Step S322) The gut score acquiring unit 149 acquires excretion-related information.

(Step S323) The gut score acquiring unit 149 selects, as learning information that is to be used, learning information corresponding to both device identifying information corresponding to the sound information and excretion-related information, out of the learning information stored in the learning information storage unit 111. That is to say, the gut score acquiring unit 149 selects, as learning information that is to be used, learning information corresponding to predetermined information contained in the life information, out of the learning information stored in the learning information storage unit 111.

This processing may be performed as follows, for example. That is to say, multiple pieces of learning information stored in the learning information storage unit 111 are set to be respectively associated with pieces of device identifying information and to be further associated with the Bristol scale, for example. This can be realized by generating learning information using multiple pieces of learning input information with the same Bristol scale. Then, in the above described processing in step S323, learning information corresponding to the device identifying information may be selected out of the learning information corresponding to the Bristol scale of the excretion-related information acquired in step S322.

(Step S324) The gut score acquiring unit 149 inputs the sound information to the learning information, and outputs an output indicator value. In this embodiment, the frequency of gut sounds contained in the abdominal sounds and a gut state estimation result are output. Subsequently, the procedure returns to the processing shown in FIG. 5.

If the abdominal sound usage processing is performed in this manner, the frequency of gut sounds and the gut state estimation result can be obtained with greater accuracy.

In the abdominal sound usage processing, learning information that is to be used may be selected based on basic information in the same manner as described above. In this case, the learning information stored in the learning information storage unit 111 may be associated with one or more elements that may be contained in the basic information, and, in the abdominal sound usage processing, learning information that is to be used may be selected based on the one or more elements in the basic information.

A third modified example is as follows. That is to say, the gut-related score acquiring unit 152 may acquire a gut-related score from sound information and life information, using learning information configured such that sound information and life information are taken as input and a gut-related score is taken as output. In this case, any element or viewpoint in the life information may be used as input information. It is also possible to acquire a gut-related score without using the life information. In either case, the basic information may be used as input information.

The learning information that is used may be generated by the learning information generating unit 147, using two or more pairs of learning input information containing sound information and life information and a gut-related score, through configuration of a classifier using the two or more pairs of information and a module for configuring a machine learning classifier, or may be generated by another apparatus or the like in the same manner.

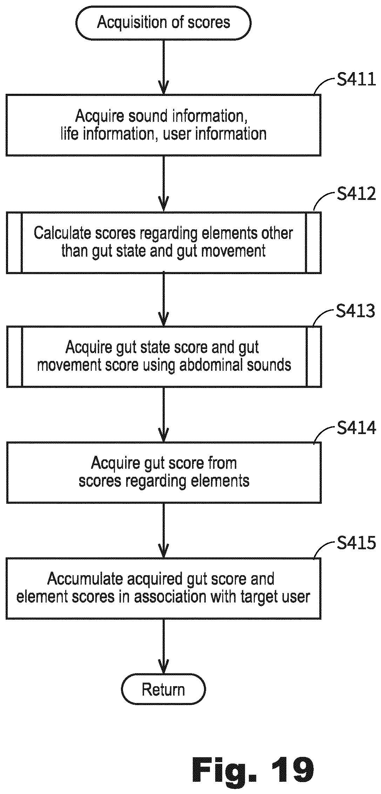

FIG. 19 is a flowchart showing an example of the score acquiring processing of the information processing apparatus 100 according to a third modified example of the embodiment of the present invention.

(Step S411) The gut score acquiring unit 149 acquires sound information, life information, past user information, and the like regarding a target user from the user information storage unit 115 based on the user identifier of the user.

(Step S412) The gut score acquiring unit 149 causes the element score acquiring unit 151 to perform processing for acquiring scores regarding elements other than the gut-related score. That is to say, the element score acquiring processing is performed for scores other than the gut-related score.

(Step S413) The gut score acquiring unit 149 performs gut-related score acquiring processing using the sound information. In this modified example, it can be said that the gut-related score acquiring processing is performed as the abdominal sound usage processing. The gut-related score acquiring processing according to this modified example will be described later in detail.

(Step S414) When the element scores are obtained through the element score acquiring processing and the gut-related score acquiring processing, the gut score acquiring unit 149 acquires a gut score from the element scores. At this time, the gut score acquiring unit 149 acquires a gut score using a predetermined method such as addition or multiplication of the element scores as described above, for example, but there is no limitation to this.

(Step S415) The gut score acquiring unit 149 accumulates the acquired gut score and element scores in the user information storage unit 115 in association with the user identifier of the target user. Subsequently, the procedure returns to the processing in FIG. 4.

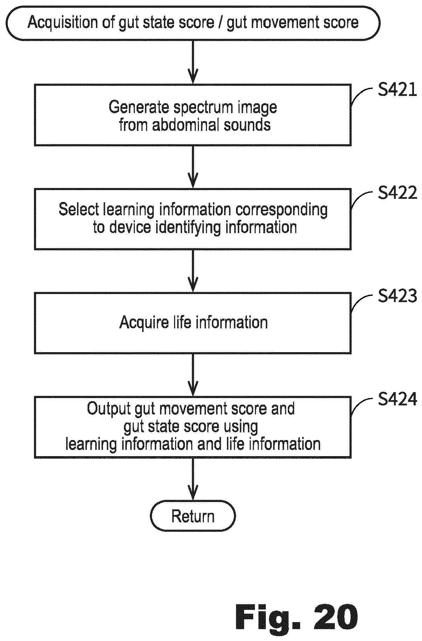

FIG. 20 is a flowchart showing an example of the gut-related score acquiring processing of the information processing apparatus 100 in the embodiment.

(Step S421) The gut score acquiring unit 149 generates a spectrum image from sound information, which is sound data obtained by recording abdominal sounds.

(Step S422) The gut score acquiring unit 149 selects, as learning information that is to be used, learning information corresponding to the device identifying information corresponding to the sound information, out of the learning information stored in the learning information storage unit 111.

(Step S423) The gut score acquiring unit 149 acquires life information. In addition, basic information may be acquired as well.

(Step S424) The gut score acquiring unit 149 inputs the sound information and the life information to the learning information, and outputs a gut movement score and a gut state score. Subsequently, the procedure returns to the processing shown in FIG. 19.

For example, if a sufficient number of pairs of learning input information and a gut-related score are available, a gut-related score and a gut score can be obtained easily and with high accuracy by directly outputting the gut-related score using the learning information generated using the information on these combinations.

If a large number of pairs of learning input information and a gut score are available, learning information in which a gut score is taken as output information may be configured using a machine learning method using these pieces of information. In this case, the gut score acquiring unit 149 may input sound information and another type of input information (life information, basic information, etc.) to the learning information, thereby acquiring a gut score. Accordingly, a gut score can be obtained easily and with high accuracy.

The storage unit 110 and the terminal storage unit 610 described above are preferably non-volatile recording media, but may alternately be realized by volatile recording media. The pieces of information respectively acquired by their corresponding apparatuses are respectively stored in these units, but there is no limitation on the procedure in which information is stored therein. For example, information and the like may be stored therein via a recording medium, information and the like transmitted via a communication line or the like may be stored therein, or information and the like input via an input device may be stored therein.

Furthermore, the processing unit 140 and the terminal processing unit 640 described above may be realized typically by MPUs, memories, or the like. Typically, the processing procedure of the processing unit 140 and the terminal processing unit 640 is realized by software, and the software is stored in a recording medium such as a ROM. The processing procedure may be realized by hardware (dedicated circuits).

Furthermore, information that can be accepted by the accepting unit 130 or the terminal accepting unit 630 may be input by any part such as a numeric keypad, a keyboard, a mouse, or a menu screen. The accepting unit 130 and the terminal accepting unit 630 may be realized by a device driver for an input part such as a numeric keypad or a keyboard, control software for a menu screen, or the like.

Furthermore, the receiving unit 120 and the terminal receiving unit 620 are typically realized by wireless or wired communication parts, but may also be realized by broadcast receiving parts.

Furthermore, the transmitting unit 170 and the terminal transmitting unit 670 are typically realized by wireless or wired communication parts, for example, but may also be realized by broadcasting parts.

The information processing apparatus 100 may be constituted by one server, multiple servers that operate in coordination with each other, or other built-in computers or the like. It will be appreciated that the server may be a so-called cloud server, an ASP server, or the like, and there is no limitation on the type thereof.

The processing in this embodiment may be realized by software. The software may be distributed by software downloads or any other suitable method. Furthermore, the software may be distributed in a form where the software is stored in a recording medium such as a CD-ROM. The software that realizes the information processing apparatus 100 in this embodiment is the following sort of program. Specifically, this program is a program for causing a computer to function as: a sound information acquiring unit that acquires sound information regarding abdominal sounds, which are sounds emanating from the abdomen, of a user; a gut score acquiring unit that acquires a gut score related to a gut condition of the user, using input information containing the sound information acquired by the sound information acquiring unit and learning information prepared in advance; and an output unit that outputs the gut score acquired by the gut score acquiring unit.

### Others

The terminal apparatuses may each have part or the whole of the configuration for realizing the functions related to the acquisition and output of gut scores as those of the information processing apparatus described above.

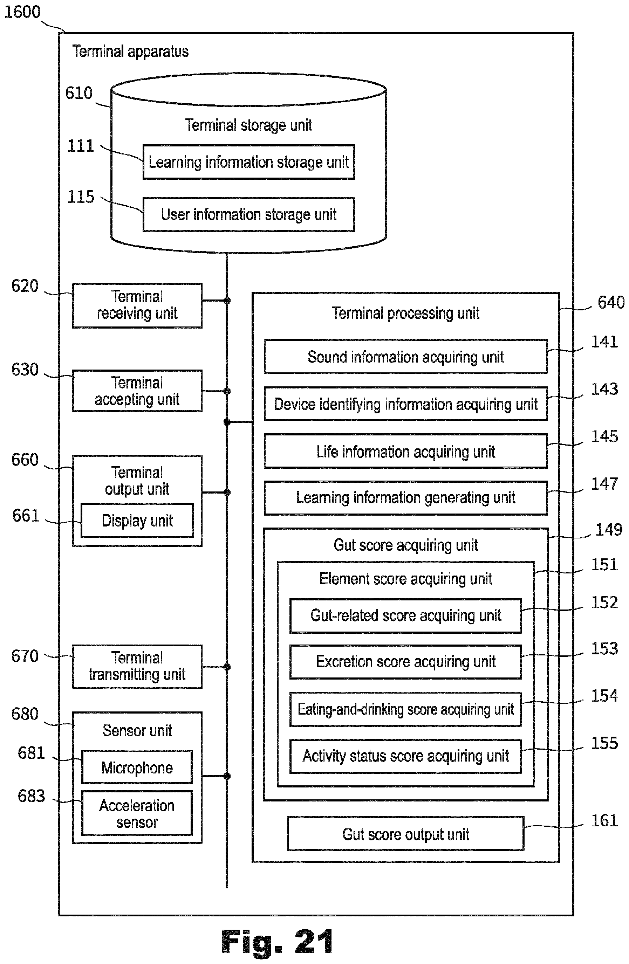

FIG. 21 is a block diagram showing the configuration of the terminal apparatus according to another modified example of the foregoing embodiment.

In FIG. 21, the constituent elements similar to those of the configuration in the foregoing embodiment are denoted by the same reference numerals. A terminal apparatus 1600 is configured to be capable of acquiring and outputting gut scores as with the information processing apparatus 100 described above.

As shown in FIG. 21, in the terminal apparatus 1600, the terminal storage unit 610 includes the learning information storage unit 111 and the user information storage unit 115. The terminal processing unit 640 includes the sound information acquiring unit 141, the device identifying information acquiring unit 143, the life information acquiring unit 145, the learning information generating unit 147, the gut score acquiring unit 149, the element score acquiring unit 151, and the gut score output unit 161. The sound information acquiring unit 141 can acquire sound information based on the abdominal sounds recorded using the microphone 681. The device identifying information acquiring unit 143 acquires device identifying information on the terminal apparatus 1600. The life information acquiring unit 145 acquires life information based on information input by the user and accepted by the terminal accepting unit 630 or information acquired using the sensor unit 680. The gut score output unit 161 causes the display unit 661 of the terminal output unit 660 to display the acquired gut score and the like, for example.

In this manner, the terminal apparatus 1600 may be configured to function as an information processing apparatus that acquires and outputs gut scores. The same effect as described above can be obtained.

With respect to the acquisition and output of gut scores, some functions and roles of the terminal apparatus 1600 may be performed or implemented by the information processing apparatus 100 or the like. For example, it is also possible to employ a configuration in which the learning information generating unit 147 is provided in the information processing apparatus 100 and the generated learning information is received and used by the terminal apparatus 1600. For example, it is also possible to employ a configuration in which the learning information storage unit 111 in which multiple pieces of learning information are stored is provided in the information processing apparatus 100 and, out of these pieces of learning information, learning information corresponding to the device identifying information and the like of the terminal apparatus 1600 is downloaded to and used by the terminal apparatus 1600.

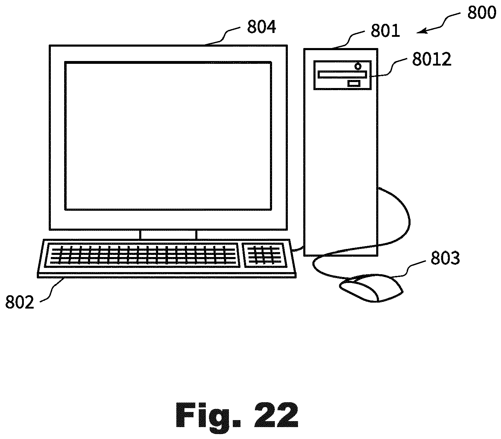

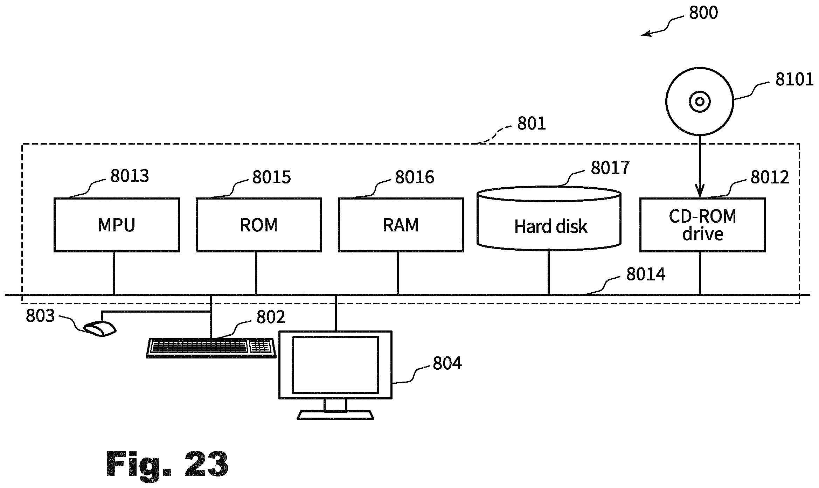

FIG. 22 is a schematic view of a computer system 800 in the foregoing embodiment. FIG. 23 is a block diagram of the computer system 800 in the embodiment.

These drawings show the configuration of a computer that executes the program described in this specification to realize the information processing apparatus and the like in the embodiment described above. The foregoing embodiment may be realized using computer hardware and a computer program executed thereon.

The computer system 800 includes a computer 801 including a CD-ROM drive, a keyboard 802, a mouse 803, and a monitor 804.

The computer 801 includes, in addition to the CD-ROM drive 8012, an MPU 8013, a bus 8014 connected to the CD-ROM drive 8012 or equivalent, a ROM 8015 in which a program such as a boot up program is stored, a RAM 8016 that is connected to the MPU 8013 and is a memory in which a command of an application program is temporarily stored and a temporary storage area is provided, and a hard disk 8017 in which an application program, a system program, and data are stored. Although not shown, the computer 801 may further include a network card that provides connection to a LAN.

The program for causing the computer system 800 to execute the functions of the information processing apparatus and the like in the foregoing embodiment may be stored in a CD-ROM 8101 that is inserted into the CD-ROM drive 8012, and be transmitted to the hard disk 8017. Alternatively, the program may be transmitted via a network (not shown) to the computer 801 and stored in the hard disk 8017. At the time of execution, the program is loaded into the RAM 8016. The program may be loaded from the CD-ROM 8101. Alternately, the program may be loaded directly from a network.

The program does not necessarily have to include, for example, an operating system (OS) or a third party program to cause the computer 801 to execute the functions of the information processing apparatus and the like in the foregoing embodiment. The program may only include a command portion to call an appropriate function (module) in a controlled mode and obtain desired results. The manner in which the computer system 800 operates is well known, and thus a detailed description thereof has been omitted.

It should be noted that, in the program, in a transmitting step of transmitting information, a receiving step of receiving information, or the like, processing that is performed only by hardware is not included. For example, processing performed by a modem or an interface card in the transmitting step (processing that can be performed only by hardware) is not included.

Furthermore, the computer that executes the program may constituted by a single computer, or constituted by multiple computers. That is to say, centralized processing may be performed, or distributed processing may be performed, respectively.

Furthermore, in the foregoing embodiment, two or more constituent elements in one apparatus may be physically realized by one medium.

Furthermore, in the foregoing embodiment, each constituent element may be configured by dedicated hardware, or alternatively, constituent elements that can be realized by software may be realized by executing a program. For example, each constituent element may be realized by a program execution unit such as a CPU reading and executing a software program stored in a recording medium such as a hard disk or a semiconductor memory. At the time of executing the program, the program execution unit may execute the program while accessing the storage unit or the recording medium. Furthermore, this program may be executed by downloading from a server or the like, or may be executed by reading a program stored in a predetermined recording medium (e.g., an optical disk, a magnetic disk, a semiconductor memory, etc.). Furthermore, the program may be used as a program for constituting a program product. Furthermore, a computer that executes the program may be a single computer or may be multiple computers. That is to say, centralized processing may be performed, or distributed processing may be performed.

In the foregoing embodiment, each process (function) may be realized as centralized processing using a single apparatus (system), or may be realized as distributed processing using multiple apparatuses (in this case, the entire system constituted by multiple apparatuses that perform distributed processing may be regarded as one "apparatus"). For example, some of the operations that are described in the foregoing embodiment as being performed by the information processing apparatus may be performed by another apparatus such as a terminal apparatus.

Furthermore, in the foregoing embodiment, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiment, information related to the processing that is performed by each constituent element, for example, information that is to be accepted, acquired, selected, generated, transmitted, or received by each constituent element, information such as a threshold value, a numerical expression, or an address used by each constituent element in the processing and the like may be retained in an unshown recording medium temporarily or for a long period of time even if not specified in the description above. Furthermore, the information may be accumulated in the unshown recording medium by each constituent element or by an unshown accumulating unit. Furthermore, the information may be read from the unshown recording medium by each constituent element or by an unshown reading unit.

Furthermore, in the foregoing embodiment, if information used by each constituent element or the like, for example, information such as a threshold value, an address, or various setting values used by each constituent element in the processing may be changed by a user, the user may be or may not be allowed to change such information as appropriate even if not specified in the description above. If the user is allowed to change such information, the change may be realized by, for example, an unshown accepting unit that accepts a change instruction from the user and an unshown changing unit that changes information according to the change instruction. The unshown accepting unit may accept the change instruction, for example, by accepting information from an input device, by receiving information transmitted via a communication line, or by accepting information read from a predetermined recording medium.

The present invention is not limited to the embodiment set forth herein. Various modifications are possible within the scope of the invention.

The configuration is not limited that described in the foregoing embodiment, and some of the constituent elements and functions of the embodiment may be omitted.

### Industrial Applicability

As described above, the information processing apparatus according to the present invention makes it possible to obtain objective information on the gut condition, thus rendering this apparatus useful as an information processing apparatus or the like.

## Claims

1. An information processing apparatus, comprising:
a sound information acquiring unit that acquires sound information regarding abdominal sounds, which are sounds emanating from the abdomen, of a user;
a gut score acquiring unit that acquires a gut score related to a gut condition of the user, using input information containing the sound information acquired by the sound information acquiring unit and learning information prepared in advance; and
a gut score output unit that outputs the gut score acquired by the gut score acquiring unit.

2. The information processing apparatus according to claim 1, wherein the input information further contains life information regarding a life state of the user.

3. The information processing apparatus according to claim 2, wherein the life information contains excretion-related information regarding an excretion status of the user.

4. The information processing apparatus according to claim 3, wherein the excretion-related information contains information indicated by Bristol Stool Form Scale input by the user.

5. The information processing apparatus according to claim 3, wherein the gut score acquiring unit further includes an excretion score acquiring unit that acquires an excretion score based on the excretion-related information, and acquires the gut score using the excretion score acquired by the excretion score acquiring unit.

6. The information processing apparatus according to claim 2, wherein the life information contains eating-and-drinking information regarding an eating-and-drinking status of the user.

7. The information processing apparatus according to claim 6, wherein the eating-and-drinking information contains at least one of information regarding the amount of water consumed, information regarding whether or not alcohol was consumed or the amount of alcohol consumed, information regarding whether or not a meal was taken or the content thereof, and information regarding whether or not a particular group of food was consumed or the amount thereof consumed.

8. The information processing apparatus according to claim 6, wherein the gut score acquiring unit further includes an eating-and-drinking score acquiring unit that acquires an eating-and-drinking score based on the eating-and-drinking information, and acquires the gut score using the eating-and-drinking score acquired by the eating-and-drinking score acquiring unit.

9. The information processing apparatus according to claim 2, wherein the life information contains activity status information regarding an activity status of the user.

10. The information processing apparatus according to claim 9, wherein the activity status information contains at least one of sleep information regarding sleep and exercise information regarding exercise.

11. The information processing apparatus according to claim 9, wherein the activity status information is information acquired by an activity tracker that acquires the level of activity of the user.

12. The information processing apparatus according to claim 9, wherein the gut score acquiring unit further includes an activity status score acquiring unit that acquires an activity status score based on the activity status information, and acquires the gut score using the activity status score acquired by the activity status score acquiring unit.

13. The information processing apparatus according to claim 1,
wherein the learning information is generated such that learning input information containing sound information is taken as information that is to be input and a value of a predetermined output indicator regarding an activity state of the guts is taken as information that is to be output, and
the gut score acquiring unit acquires the gut score using the value of the output indicator acquired using the learning information.

14. The information processing apparatus according to claim 13, wherein the output indicator is at least one of a bowel movement state and the number of peristalsis movements of the guts per unit time.

15. The information processing apparatus according to claim 1,
wherein the gut score acquiring unit includes an element score acquiring unit that acquires element scores respectively for two or more evaluation elements based on the input information, and acquires the gut score using the element scores acquired by the element score acquiring unit, and
the output unit further outputs a radar chart using the element scores acquired by the element score acquiring unit.

16. The information processing apparatus according to claim 1, further comprising:
a device identifying information acquiring unit that acquires device identifying information for identifying the type of device used to acquire abdominal sounds corresponding to the sound information,
wherein multiple pieces of learning information are each prepared in association with device identifying information, and
the gut score acquiring unit acquires the gut score using the learning information corresponding to the device identifying information acquired by the device identifying information acquiring unit.

17. The information processing apparatus according to claim 1, further comprising:
a microphone for recording the abdominal sounds; and
a display unit that displays the gut score output by the output unit.

18. An information processing method realized using a sound information acquiring unit, a gut score acquiring unit, and an output unit, comprising:
a sound information acquiring step of the sound information acquiring unit acquiring sound information regarding abdominal sounds, which are sounds emanating from the abdomen, of a user;
a gut score acquiring step of the gut score acquiring unit acquiring a gut score related to a gut condition of the user, using input information containing the sound information acquired in the sound information acquiring step and learning information prepared in advance; and
an output step of the output unit outputting the gut score acquired in the gut score acquiring step.

19. A recording medium on which a program is recorded, the program causing a computer to function as:
a sound information acquiring unit that acquires sound information regarding abdominal sounds, which are sounds emanating from the abdomen, of a user;
a gut score acquiring unit that acquires a gut score related to a gut condition of the user, using input information containing the sound information acquired by the sound information acquiring unit and learning information prepared in advance;
an output unit that outputs the gut score acquired by the gut score acquiring unit.
